# EUROPEAN PATENT APPLICATION

(11) **EP 4 137 556 A1**
(43) Date of publication of application: **22.02.2023**
(21) Application number: 21787958.4
(22) Date of filing: 12.04.2021
(51) Int. Cl.: C12M 1/40, C12P 7/04, C12N 11/14

(54) **MICROBIAL GAS-PHASE REACTION**

(30) Priority: 13.04.2020 JP 2020071961
(71) Applicant: National University Corporation Tokai National Higher Education and Research System, Nagoya-shi, Aichi 464-8601 (JP)
(72) Inventor: HORI, Katsutoshi, Nagoya-shi, Aichi 464-8601 (JP); CHEN, Yanyu, Nagoya-shi, Aichi 464-8601 (JP)
(74) Representative: Engelhard, Markus
(86) International application number: PCT/JP2021/015227
(87) International publication number: WO 2021/210553

(57) **Abstract**

The present disclosure provides a conversion of a gaseous substrate with the use of a microorganism. In one aspect, the present disclosure provides a microbial gas-phase reaction system for converting a gaseous substrate with the use of a microorganism. This microbial gas-phase reaction system comprises at least one member selected from among a carrier having the microorganism immobilized thereon, a gas supply part for supplying the gaseous substrate to the gas phase of the microbial gas-phase reaction system, and a water supply system for supplying water to the carrier. In one aspect, the present disclosure provides a method for converting a gaseous substrate with the use of a microorganism. This method comprises a step for exposing a surface of a carrier, on which the microorganism is immobilized, to a gas phase containing the gaseous substrate.

## Description

### [Technical Field]

The present disclosure relates to conversion of a gaseous substrate by a microorganism.

### [Background Art]

Microorganisms can cause a chemical reaction to proceed under a mild condition through various enzyme reactions without the need for a large-scale facility as compared to a chemical reaction using a physicochemical method. Thus, compound production utilizing microorganisms is being studied.

When a gaseous substrate is converted using a microorganism, it is necessary to provide much energy for aeration, stirring and the like in supplying the substrate. In addition, since a hydrophobic gaseous substance has a low dissolution efficiency, there is a problem that such a substance also has a low reaction efficiency.

### [Summary of Invention]

### [Solution to Problem]

As a result of diligent studies, the present inventors achieved efficient and/or stable conversion of a gaseous substrate using a microorganism. Thus, the present disclosure provides a method for converting a gaseous substrate using a microorganism and a gas phase microorganism reaction system for converting a gaseous substrate using a microorganism.

Therefore, the present disclosure provides the following.

### (Item 1)

A gas phase microorganism reaction system comprising:
a gas phase reaction chamber comprising a gas supply section which continuously or intermittently supplies a gaseous substrate from outside;
a carrier which is installed in the gas phase reaction chamber or a carrier which constitutes at least a part of a wall of the gas phase reaction chamber, wherein the carrier is moisture-permeable or water-absorbent, but put in a state where any part of a surface on the gas phase side is not immersed in water, wherein a microorganism cell is immobilized to the carrier such that at least a part of the microorganism cell is in direct contact with a gas phase in the gas phase reaction chamber; and
a gas discharge section which is connected to the gas phase reaction chamber.

### (Item 2)

The gas phase microorganism reaction system of item 1, further comprising a moisture supply system for supplying moisture from outside the gas phase reaction chamber to the extent without drying up of the microorganism cell that is immobilized to the carrier.

### (Item 3)

The gas phase microorganism reaction system of any of the preceding items, wherein the moisture supply system is configured such that a water content of the carrier is 1 to 50% (v/v).

### (Item 4)

The gas phase microorganism reaction system of any of the preceding items, further comprising a mechanism which collects a product resulting from conversion of the gaseous substrate by the microorganism.

### (Item 5)

The gas phase microorganism reaction system of any of the preceding items, wherein the gas supply section is configured to passively take in the gaseous substrate by diffusion from outside the gas phase reaction chamber.

### (Item 6)

The gas phase microorganism reaction system of any of the preceding items, wherein the carrier is a thin membrane.

### (Item 7)

The gas phase microorganism reaction system of any of the preceding items, further comprising a liquid discharge section which is connected to the gas phase reaction chamber.

### (Item 8)

The gas phase microorganism reaction system of any of the preceding items, wherein the moisture supply system is configured to supply the moisture to the carrier as a liquid flow.

### (Item 9)

The gas phase microorganism reaction system of any of the preceding items, wherein the moisture supply system comprises a water phase chamber in which liquid flows, wherein the carrier is disposed between the water phase chamber and the gas phase reaction chamber, and wherein the carrier is disposed such that a surface of the carrier on which the microorganism is immobilized faces the gas phase reaction chamber side.

### (Item 9A)

The gas phase microorganism reaction system of any of the preceding items, wherein the carrier is configured to separate the gas phase chamber from the water phase chamber.

### (Item 9B)

The gas phase microorganism reaction system of any of the preceding items, wherein a flow channel from supply to discharge of the gaseous substrate is configured to be spiral.

### (Item 9C)

The gas phase microorganism reaction system of any of the preceding items, wherein a flow channel from supply to discharge of the gaseous substrate is configured to be along a carrier surface.

### (Item 10)

The gas phase microorganism reaction system of any of the preceding items, wherein the reaction vessel comprises a plurality of gas phases and a plurality of liquid phases, the gas phases and the liquid phases being configured to be alternately layered.

### (Item 11)

The gas phase microorganism reaction system of any of the preceding items, wherein the carrier comprises a plurality of tubular members, wherein the plurality of tubular members are bundled in close contact with each other to form a columnar unit, or the plurality of tubular members are disposed at an interval from each other in an outer tube to form a columnar unit comprising the plurality of tubular members and the outer tube, and wherein the plurality of tubular members and the outer tube constitute a wall of the gas phase reaction chamber or the water phase chamber.

### (Item 12)

The gas phase microorganism reaction system of any of the preceding items, further comprising a rotation mechanism which is connected to the carrier, wherein the rotation mechanism comprises a rotation axis to which the carrier is immobilized.

### (Item 13)

The gas phase microorganism reaction system of any of the preceding items, wherein the moisture supply system is configured to supply the moisture to the carrier by a capillary phenomenon.

### (Item 14)

The gas phase microorganism reaction system of any of the preceding items, wherein the moisture supply system is configured to supply the moisture to the carrier in an aerosol state or a gas state.

### (Item 15)

The gas phase microorganism reaction system of any of the preceding items, wherein the moisture supply system is configured such that the moisture in a gas state is condensed on the carrier.

### (Item 16)

The gas phase microorganism reaction system of any of the preceding items, further comprising a power source for driving the rotation mechanism.

### (Item 17)

A method for converting a gaseous substrate by a microorganism, the method comprising:
continuously or intermittently supplying a gaseous substrate from outside to a gas phase reaction chamber; and
exposing, in the gas phase reaction chamber, at least a part of a surface on which the microorganism is immobilized on the carrier to a gas phase in the gas phase reaction chamber.

### (Item 18)

The method of any of the preceding items, further comprising supplying moisture to a microorganism loaded carrier from outside the gas phase reaction chamber.

### (Item 19)

The method of any of the preceding items, which controls a water content of the carrier to 1 to 50%.

### (Item 20)

A kit comprising:
a carrier for immobilizing a microorganism or a carrier to which a microorganism is immobilized;
a gas phase reaction chamber or a gas phase reaction chamber member configured to be assembled to provide the gas phase reaction chamber;
a gas supply section which continuously or intermittently supplies a gaseous substrate from outside; and
a gas discharge section,
wherein the gas phase reaction chamber may be provided in a state where the gas phase reaction chamber comprises at least one of the carrier, the gas supply section, and the gas discharge section, or may be provided separately from the carrier, the gas supply section, and the gas discharge section,
wherein the gas phase reaction chamber is configured to contain the carrier, and
wherein the gas phase reaction chamber is configured such that a microorganism immobilized to the carrier is in contact with the gaseous substrate.

### (Item 21)

The kit of any of the preceding items, further comprising a moisture supply system for supplying moisture to the carrier from outside the gas phase reaction chamber.

### (Item 22)

The kit of any of the preceding items, further comprising a support which is configured to support the carrier.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Since the present disclosure can provide efficient and/or stable conversion of a gaseous substrate using a microorganism, the present disclosure can be advantageously used for removal of an unnecessary gaseous substance and/or acquisition of a desired product. Further, the conversion of a gaseous substrate according to the present disclosure can provide an efficient, stable, energy-saving gas phase microorganism reaction system.

### [Brief Description of Drawings]

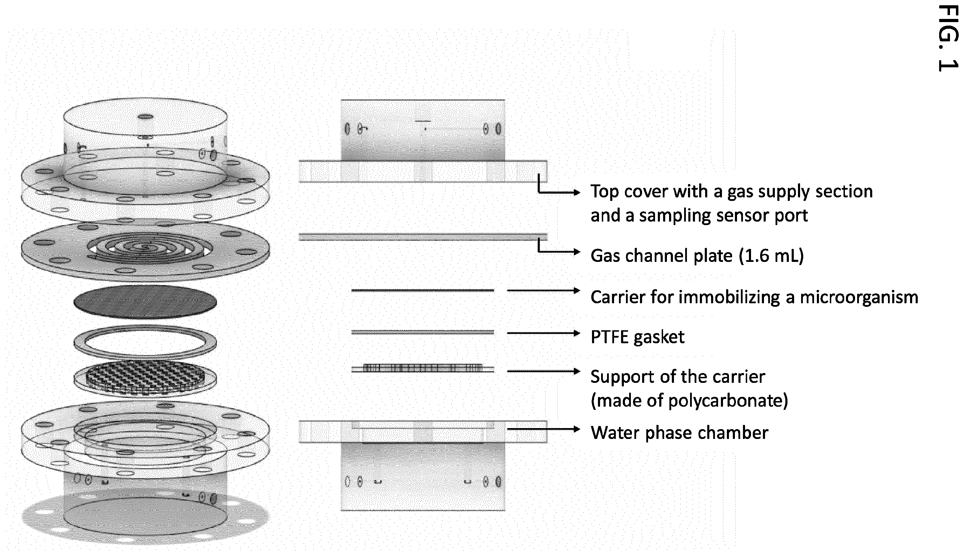

[Figure 1] Figure 1 is a specific configuration example of the bioreactor (gas phase microorganism reaction system) of the present disclosure.

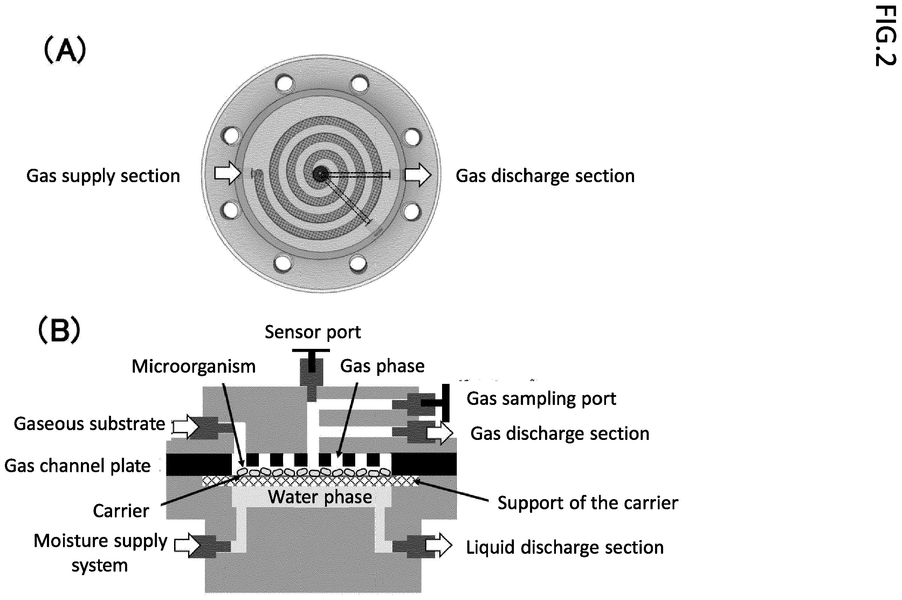

[Figure 2] (A) Figure **2A** is an overhead view of the bioreactor of Figure **1** seen from the top, which shows a configuration wherein gas is supplied from a line on one side, and gas is discharged from a line on the other side. (B) Figure **2B** is a cross-sectional view of the bioreactor of Figure **1** seen from a side surface.

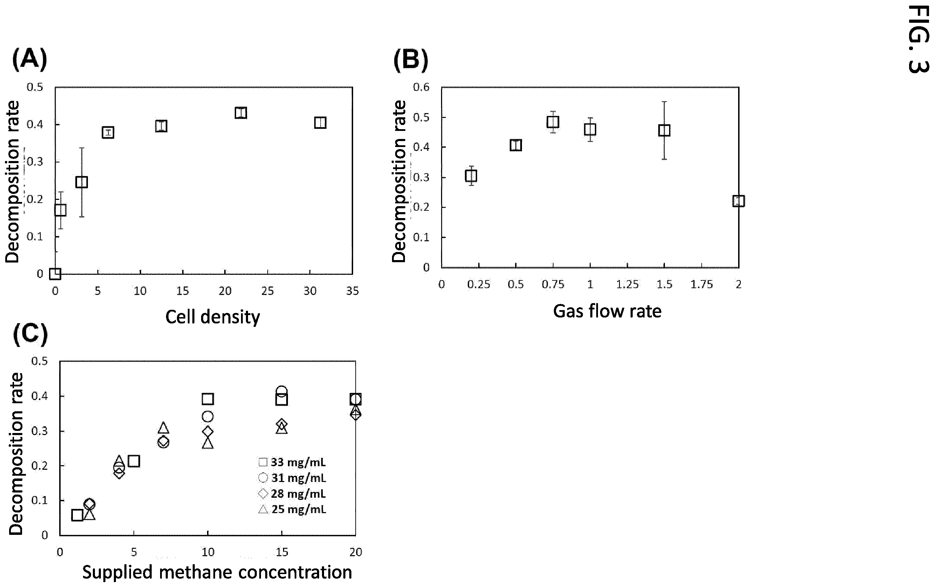

[Figure 3] Figure **3** shows methane continuous decomposition by the bioreactor of Figure **1** using Methylococcus capsulatus (Bath). The vertical axis indicates the methane decomposition rate (mLₘₑₜₕₐₙₑ/hr/mL_{gas phase}). (A) Figure **3A** shows the result from supplying air comprising 20% methane at a flow rate of 0.5 mL/min. The horizontal axis indicates the cell density (dry weight g/L_{gas phase}). (B) Figure **3B** shows the result from supplying air comprising 20% methane and using a microorganism at a cell density of 33 g - dry weight/L_{gas phase}. The horizontal axis indicates the gas flow rate (mL/min). (C) Figure **3C** shows the result from supplying air comprising 20% methane at a flow rate of 0.5 mL/min. The immobilized microorganism concentration which was used is 33, 31, 28, or 25 g - dry weight/L_{gas phase} as indicated by each symbol in the graph. The horizontal axis indicates the supplied methane concentration (v/v%).

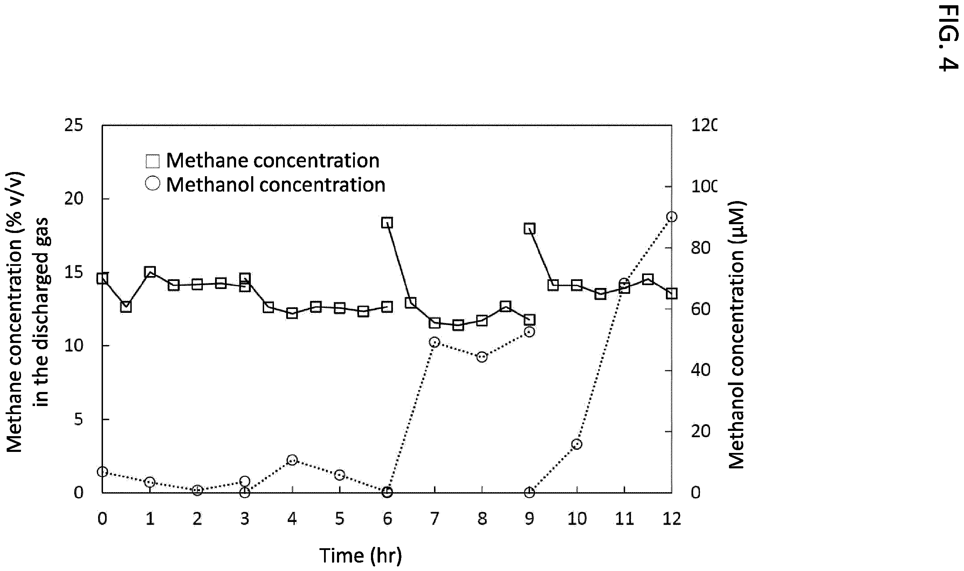

[Figure 4] Figure **4** shows the result of methane decomposition and methane → methanol conversion when air comprising 20% methane was supplied to the bioreactor of Figure **1** using Methylococcus capsulatus (Bath) at a flow rate of 0.1 mL/min. Figure **4** shows methane decomposition and methanol production when the composition of the supplied liquid was changed at each stage of 0 to 3 hours (12.9 mM phosphate buffer), 3 to 6 hours (0.1 × nitrate mineral salt (NMS) medium + 10 mM formic acid), 6 to 9 hours (0.1 × nitrate mineral salt (NMS) medium + 10 mM formic acid + 1 µM cyclopropanol), and 9 to 12 hours (0.1 × nitrate mineral salt (NMS) medium + 10 mM formic acid + 10 µM cyclopropanol). The vertical axis indicates the residual methane concentration (% v/v) in the discharged gas and the accumulated methanol concentration (µM) in the liquid phase chamber. The horizontal axis indicates the time (hr).

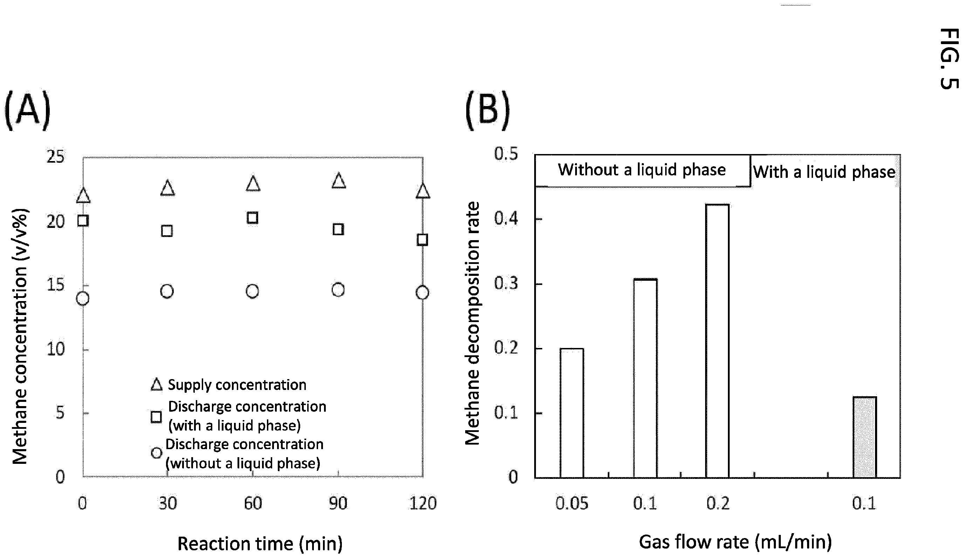

[Figure 5] Figure **5** shows that the presence of a liquid phase in the bioreactor of Figure **1** affects the rate of decomposing methane by Methylococcus capsulatus (Bath). (A)
Figure **5A** shows the results from supplying air comprising 22.8% (v/v) methane at a flow rate of 0.1 mL/min. The horizontal axis indicates the reaction time. The vertical axis indicates each of the methane concentration in the gas supply section (Δ), the methane concentration in the gas discharge section of when no water was placed in the water phase chamber to attain a gas phase (o), and the methane concentration in the gas discharge section of when water was placed in the water phase chamber (□). (B) Figure **5B** shows the results from supplying air comprising 22.8% (v/v) methane at a flow rate of 0.05, 0.1, or 0.2 mL/min and attaining a gas phase without placing water in the water phase chamber, and supplying air comprising 22.8% (v/v) methane at a flow rate of 0.1 mL/min and placing water in the water phase chamber. The vertical axis indicates the methane decomposition rate (mLₘₑₜₕₐₙₑ/hr/mL_{gas phase}).

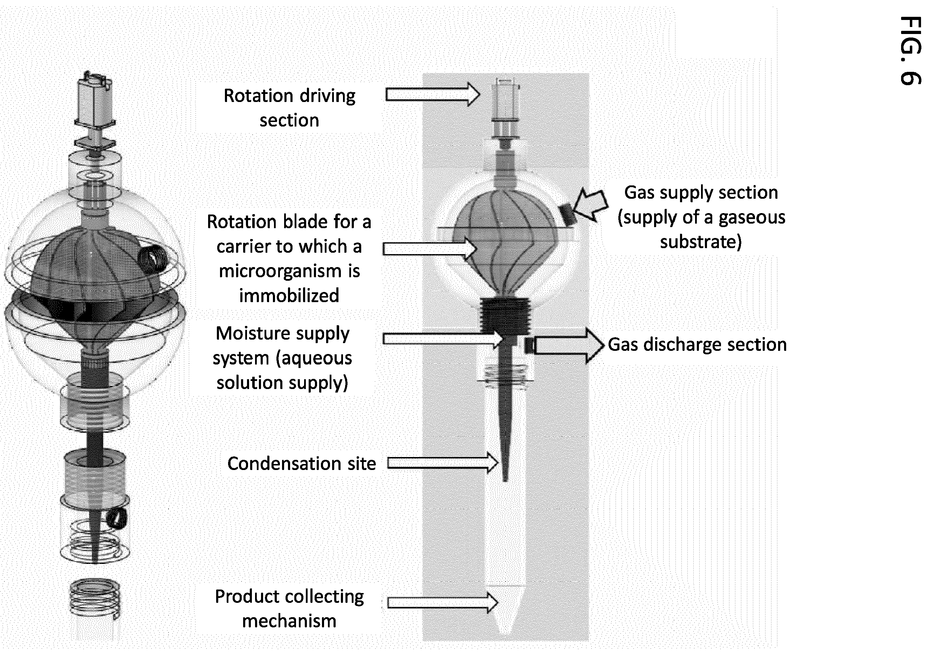

[Figure 6] Figure **6** is a specific configuration example of the bioreactor (gas phase microorganism reaction system) of the present disclosure.

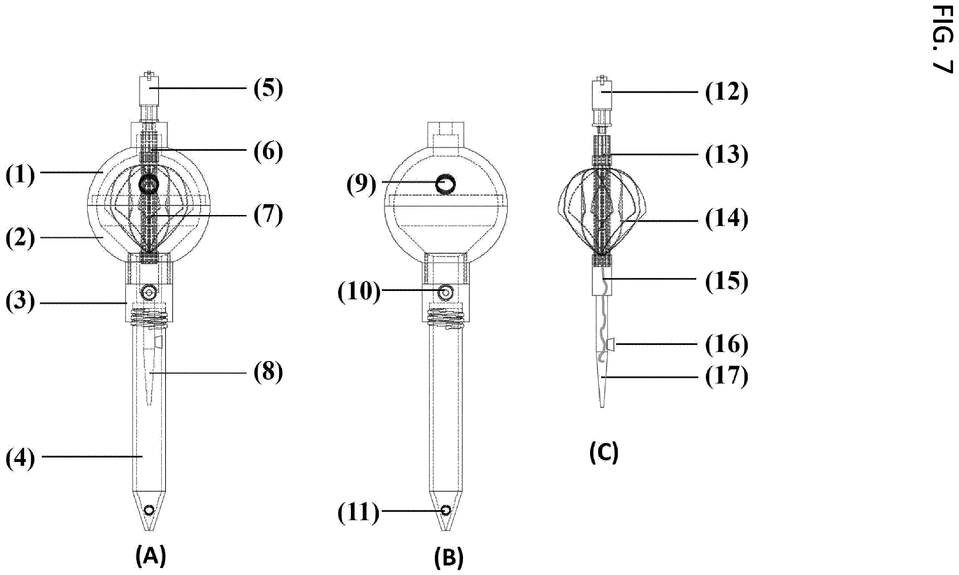

[Figure 7] (A) Figure **7A** is a side view of the combined outer unit and inner unit of the bioreactor of Figure **6****,** which is one embodiment of the gas phase reaction chamber of the present disclosure. (1): A top cover (comprising a port for infusing a gaseous substrate and water vapor) of the bioreactor. (2): A bottom cover of the bioreactor. (3): A connector for assembling **(2)** and **(4).** (4): A liquid collecting chamber. (5): A motor (movement mechanism) for rotating the inner unit. (6): A bundle of rod-like members for supporting **(7)** (a support of a carrier). (7): A fan (carrier) composed of a carrier to which a microorganism is immobilized. (8): Aqueous solution. (B) Figure **7B** is a side view of the outer unit of the bioreactor of Figure **6****.** (9): A port for infusing a gaseous substrate and water vapor (gas supply section and moisture supply system). (10): A port for discharging and/or sampling gas (gas discharge section). (11): A port for discharging and/or sampling product-containing liquid (product collecting mechanism). (C) Figure **7C** is a side view of the inner unit of the bioreactor of Figure **6****.** (12): A motor for rotating the inner unit (corresponding to **(5)** of **(A)**). (13): A bundle of rod-like members for supporting **(14)** (corresponding to **(6)** of **(A)**). (14) : A fan composed of a carrier to which a microorganism is immobilized (corresponding to **(7)** of **(A)**). (15): A member which supplies moisture to the carrier by utilizing a capillary phenomenon (a part of the moisture supply system). (16): An aqueous solution supply opening (a part of the moisture supply system). (17): Aqueous solution (corresponding to **(8)** of **(A)**).

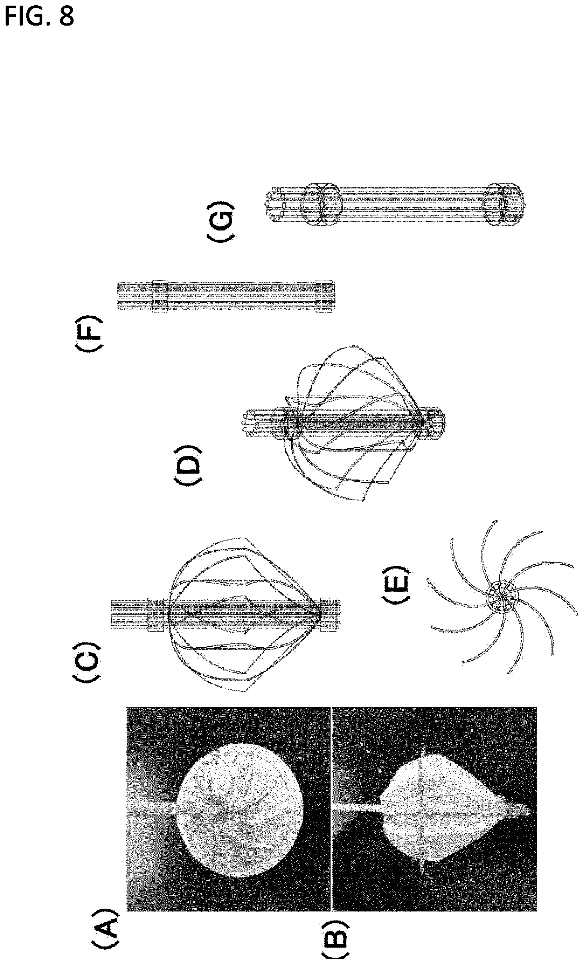

[Figure 8] Figure **8** is a specific example of the fan partof the inner unit of the bioreactor of Figure 6. (A) Figure **8A** is a picture from the top. (B) Figure **8B** is a picture from a side surface. (C) Figure **8C** is a side view. (D)
Figure **8D** is a perspective view. (E) Figure **8E** is an overhead view. (F) Figure **8F** is a side view of the axis of the fan part. (G) Figure **8G** is a perspective view of the axis of the fan part.

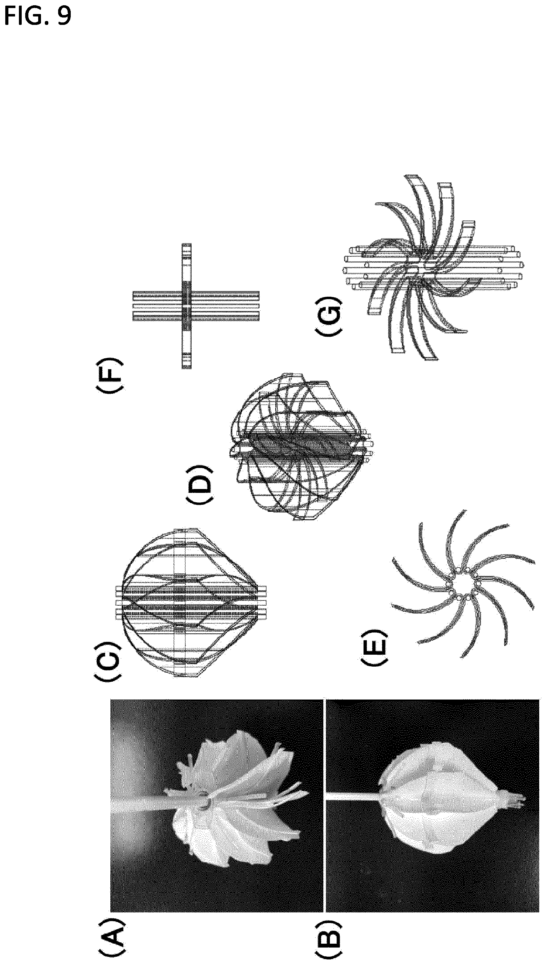

[Figure 9] Figure **9** is a specific example of the fan part of the inner unit of the bioreactor of Figure **6****.** (A) Figure **9A** is a picture from the top. (B) Figure **9B** is a picture from a side surface. (C) Figure **9C** is a side view. (D) Figure **9D** is a perspective view. (E) Figure **9E** is an overhead view. (F) Figure **9F** is a side view of the axis of the fan part. (G) Figure **9G** is a perspective view of the axis of the fan part.

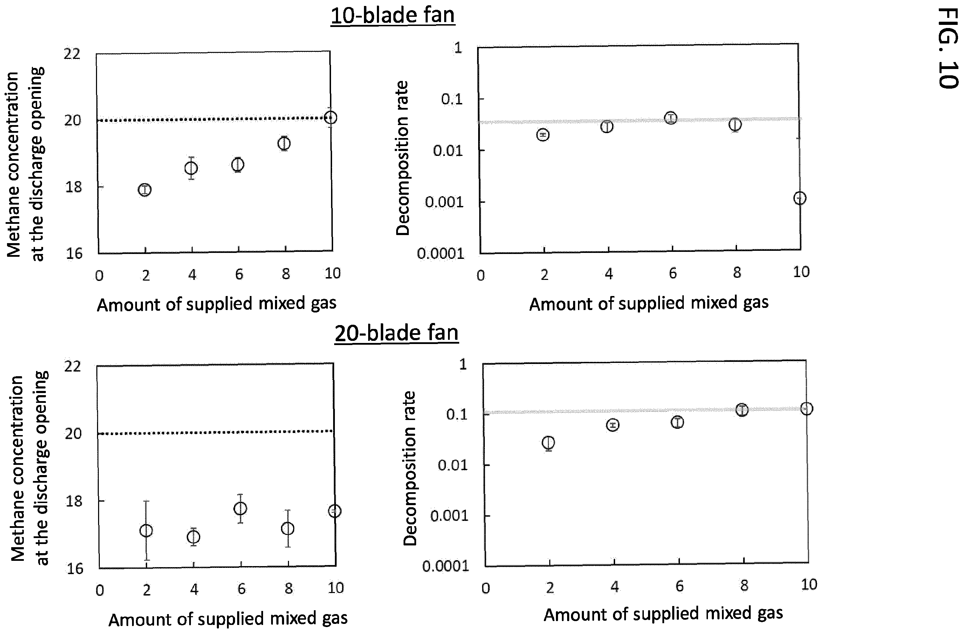

[Figure 10] Figure **10** shows methane continuous decomposition by the bioreactor of Figure **6** using Methylococcus capsulatus (Bath). The top row shows the results from using a bioreactor comprising a 10 blade fan (dry cell weight of 1.4 g). The bottom row shows the results from using a bioreactor comprising a 20 blade fan (dry cell weight of 1.3 g). The horizontal axis of each graph indicates the amount of supplied mixed gas of methane and air (mL/min). The vertical axis of the graphs on the left side indicates the methane concentration at the discharge opening (v/v%). The vertical axis of the graphs on the right side indicates the methane decomposition rate (mLₘₑₜₕₐₙₑ/hr/mL_{gas phase}).

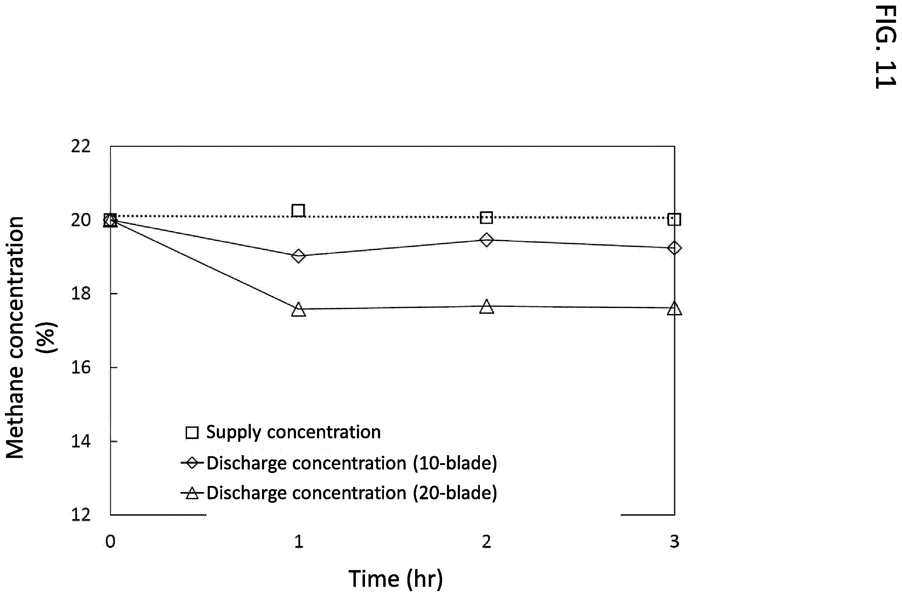

[Figure 11] Figure **11** shows the results of methane decomposition in the bioreactor of Figure **6** using Methylococcus capsulatus (Bath). These are results from supplying air comprising 20% (v/v) methane at a flow rate of 8 mL/min. The horizontal axis indicates the reaction time. The vertical axis indicates each of the methane concentration in the gas supply section (□), the methane concentration in the gas discharge section of when a 10 blade fan was used (◊), and the methane concentration in the gas discharge section of when a 20 blade fan was used (Δ).

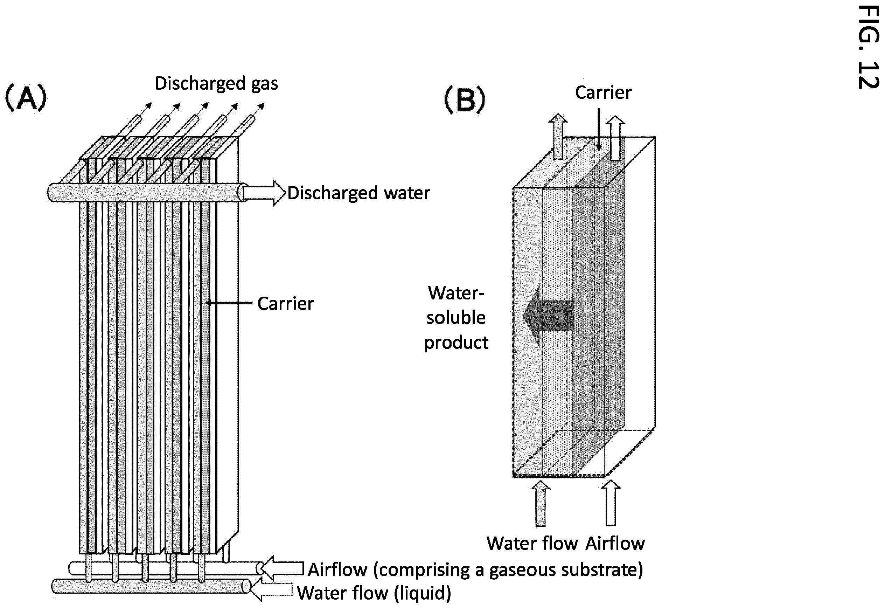

[Figure 12] (A) Figure **12A** is a specific configuration example of a part of the bioreactor (gas phase microorganism reaction system) of the present disclosure in which gas phases and liquid phases are layered. (B) Figure **12B** shows one unit of the bioreactor of **(A),** wherein a carrier is installed between a gas phase and a liquid phase.

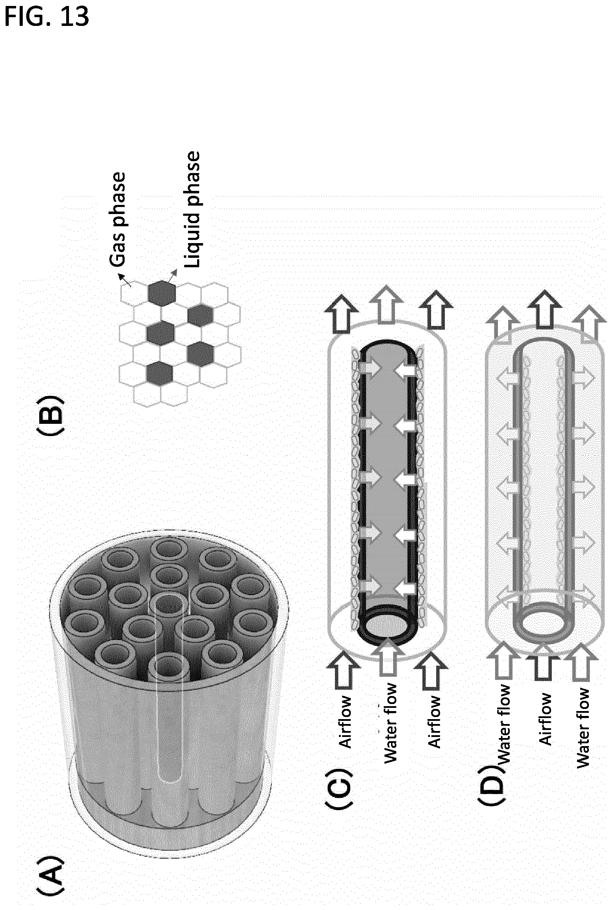

[Figure 13] (A) Figure **13A** is a specific configuration example of a part of the bioreactor (gas phase microorganism reaction system) of the present disclosure comprising a carrier including a plurality of tubular members. The plurality of tubular members are disposed at an interval from each other in an outer tube, and the plurality of tubular members and the outer tube form a columnar unit. In one embodiment, the inside of the plurality of tubular members functions as a water phase chamber while the outside of the plurality of tubular members and the inside of the outer tube function as a gas phase reaction chamber. In another embodiment, the inside of the plurality of tubular members functions as a gas phase reaction chamber while the outside of the plurality of tubular members and the inside of the outer tube function as a water phase chamber. (B) Figure **13B** shows a carrier in which a plurality of tubular members with a hexagonal cross section (honeycomb) are bundled in close contact with each other to form a columnar unit. Figure **13B** shows that the inside of some tubular members functions as a water phase chamber while the inside of other tubular members functions as a gas phase reaction chamber. (C)
Figure **13C** shows one embodiment of the operation of the bioreactor of **(A),** wherein a liquid phase is formed inside a tubular member while a gas phase is formed outside the tubular member. (D) Figure **13D** shows one embodiment of the operation of the bioreactor of **(A),** wherein a gas phase is formed inside a tubular member while a liquid phase is formed outside the tubular member.

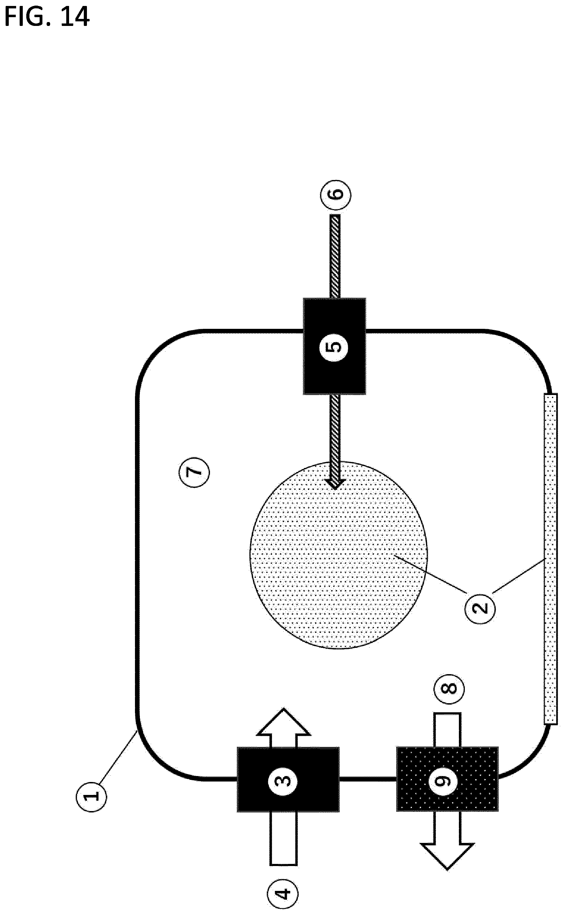

[Figure 14] Figure **14** is a schematic view of an exemplary embodiment of the gas phase microorganism reaction system of the present disclosure. (1) A gas phase reaction chamber, (2) a carrier (which is installed in the gas phase reaction chamber and/or constitutes at least a part of the wall of the gas phase reaction chamber), (3) a gas supply section, (4) a gaseous substrate, (5) a moisture supply system (as needed), (6) moisture (as needed), (7) space in the gas phase reaction chamber comprising a gas phase, (8) gas, and (9) a gas discharge section.

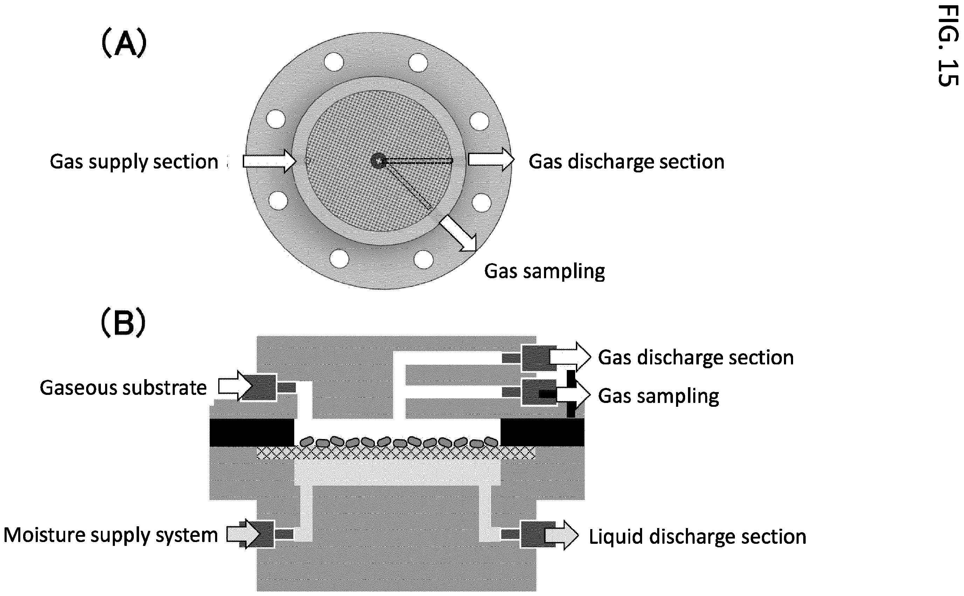

[Figure 15] (A) Figure **15A** is an overhead view of the bioreactor of Example 6 seen from the top, which shows a configuration wherein gas is supplied from a line on one side, and gas is discharged from a line on the other side. (B) Figure **15B** is a cross-sectional view of the bioreactor of Example 6 seen from a side surface.

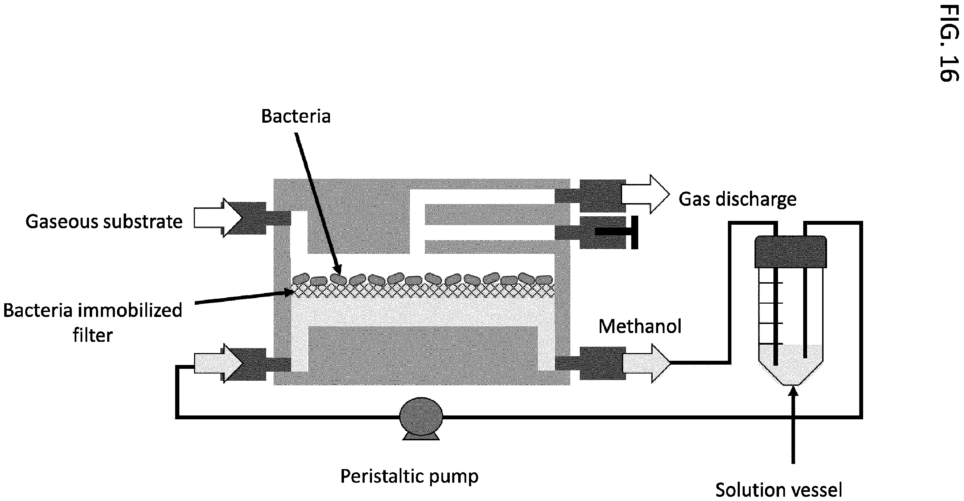

[Figure 16] Figure **16** is a schematic view of the bioreactor of Example 6 when the bioreactor was operated while disposing a methane assimilating bacteria immobilized filter between a gas phase and a liquid phase such that the face on which the bacteria were immobilized would be on the gas phase side.

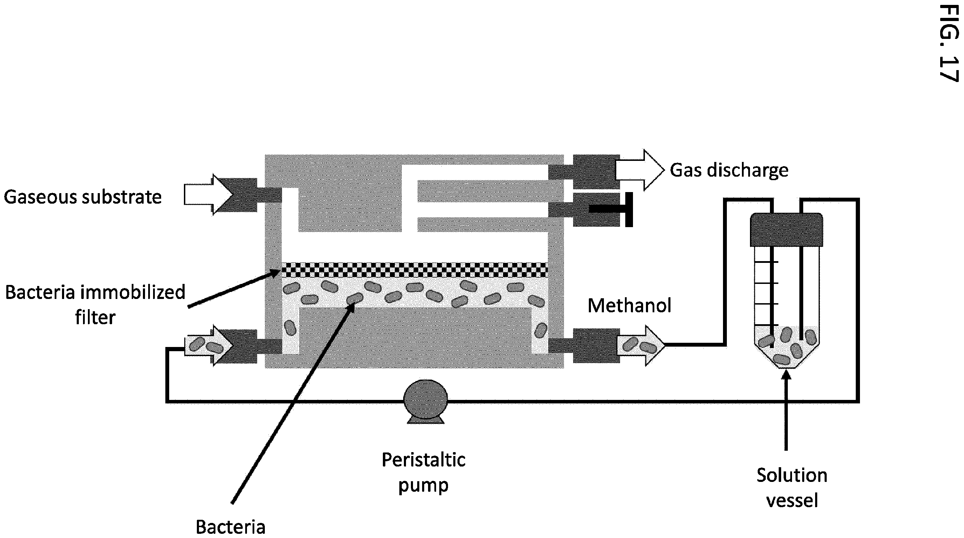

[Figure 17] Figure **17** is a schematic view of the bioreactor of Example 6 when the bioreactor was operated while disposing a methane assimilating bacteria immobilized filter between a gas phase and a liquid phase such that the face on which the bacteria were immobilized would be on the liquid phase side.

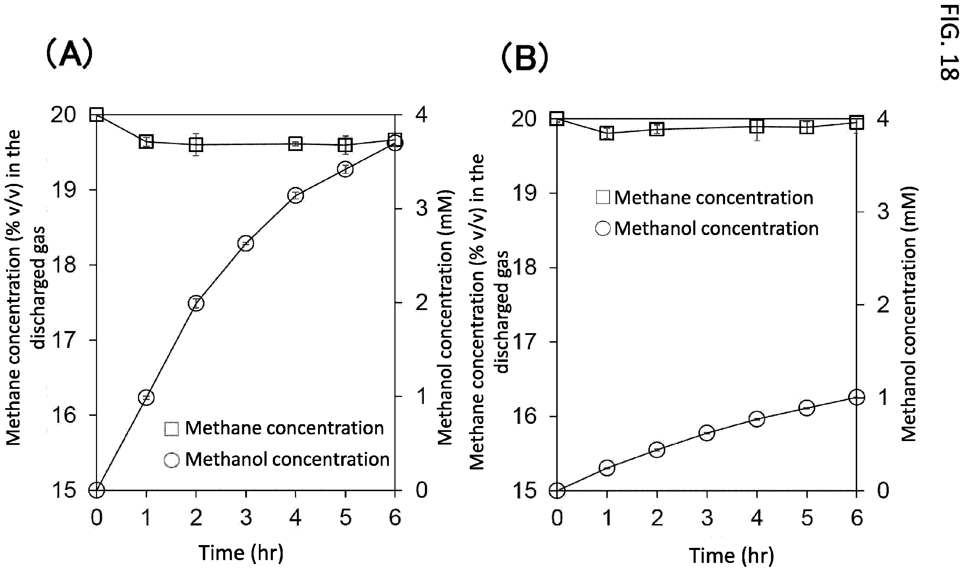

[Figure 18] Figure **18** shows the result of methane → methanol conversion when the filter was disposed such that the face on which the bacteria were immobilized would be on the gas phase side **(A)** or on the liquid phase side **(B).** The vertical axes indicate the residual methane concentration (% v/v) in the discharged gas and the accumulated methanol concentration (mM) in the liquid phase chamber. The horizontal axis indicates the time (hr).

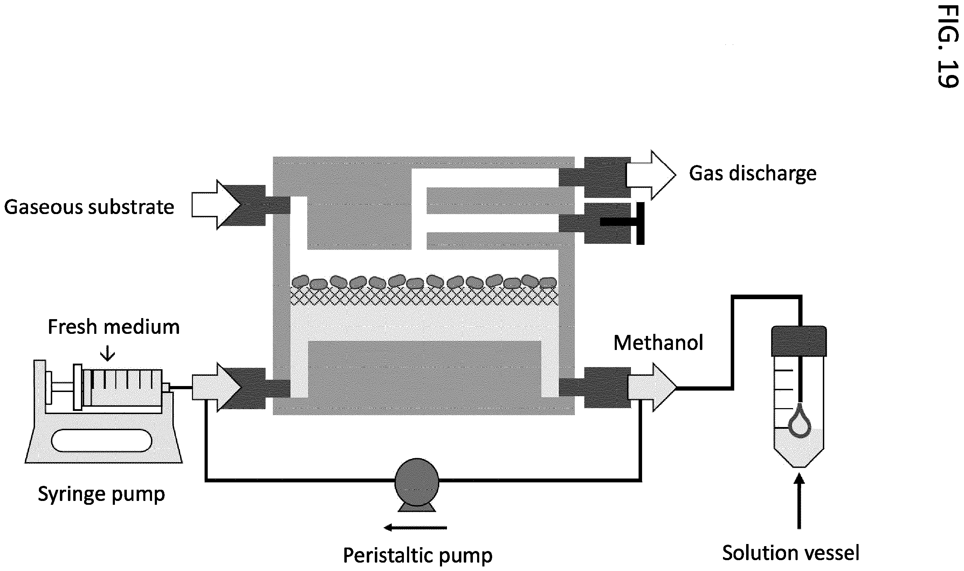

[Figure 19] Figure **19** is a schematic view of the bioreactor of Example 6 when the bioreactor was continuously operated for a long period of time while disposing a methane assimilating bacteria immobilized filter between a gas phase and a liquid phase such that the face on which the bacteria were immobilized would be on the gas phase side.

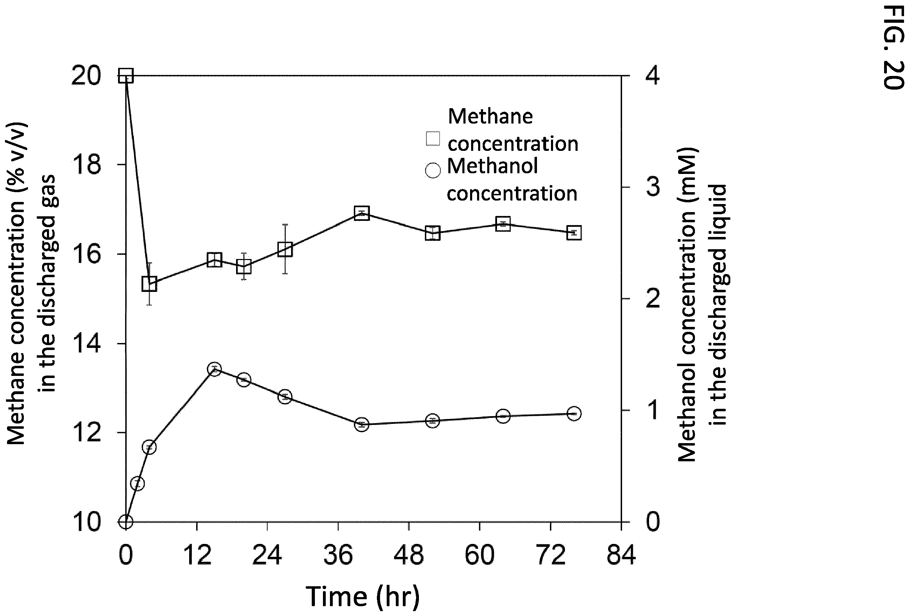

[Figure 20] Figure **20** shows the result of methane → methanol conversion for a long period of time in the bioreactor of Example 6. The vertical axis indicates the residual methane concentration (% v/v) in the discharged gas and the methanol concentration (mM) in the discharged liquid extracted from the liquid phase. The horizontal axis indicates the time (hr).

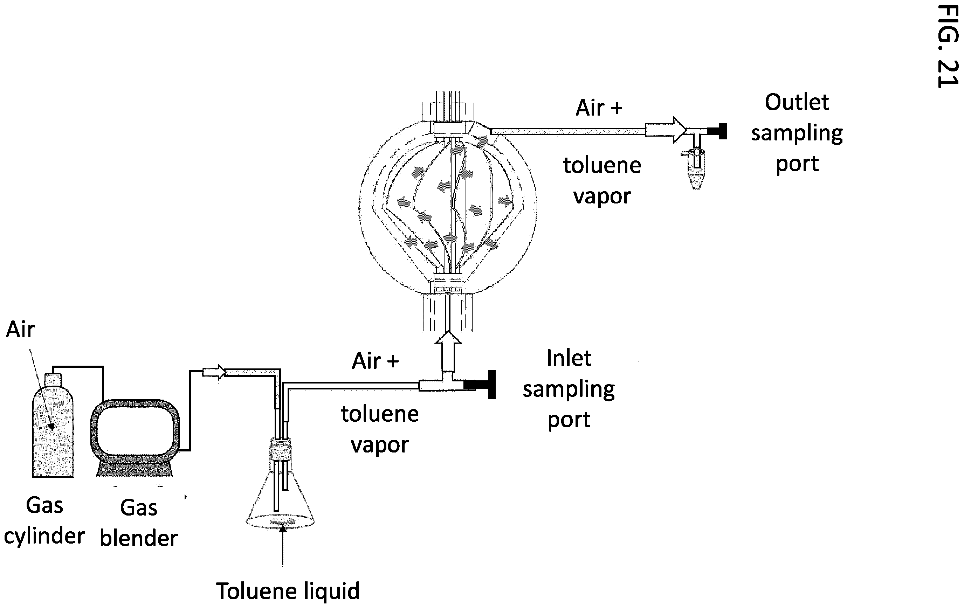

[Figure 21] Figure **21** is a schematic view of the system used in Example 7.

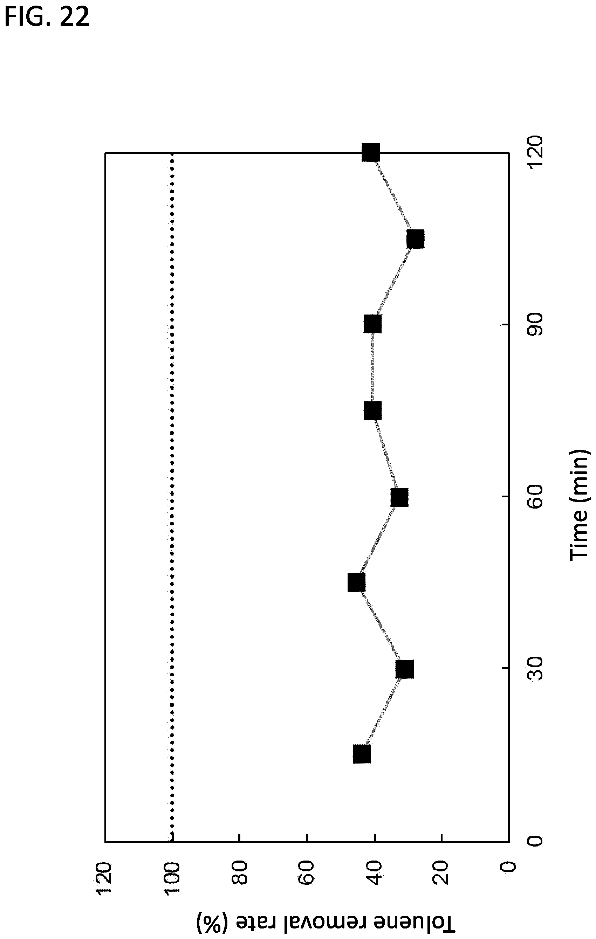

[Figure 22] Figure **22** shows the result of toluene decomposition in the bioreactor of Example 7. The vertical axis indicates the toluene removal rate (%) at the outlet of the bioreactor. The horizontal axis indicates the time (min) .

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or basic technical concepts that are particularly used herein are described hereinafter when appropriate.

### (Definitions, etc.)

As used herein, "microorganism" means an organism including microalga, yeast, and moss in addition to eubacteria and archaebacteria.

As used herein, "substrate" refers to any substance which is utilized by a microorganism or an enzyme secreted by a microorganism, and typically refers to a substance which is converted into another substance.

As used herein, "conversion" of a substrate by a microorganism refers to changing a substrate substance into another substance. In one embodiment, conversion is intended as producing another substance that has been changed as a product, and collecting this product. In one embodiment, conversion is decomposition, which is not intended as collection of another substance that has been changed.

As used herein, "product" of a microorganism refers to any substance produced by the microorganism, and includes a cell itself, an intracellular component, an extracellular component, a cell membrane, a cell wall, a secretion and the like of the microorganism. A preparation obtained by subjecting them to an operation such as crushing or purification is also included.

As used herein, "water content" refers a volume ratio of water in an object. A water content can be measured by any suitable method. For example, a water content can be calculated by calculating the volume of absorbed moisture based on the specific gravity from a difference in the weight between before and after drying the object and dividing said volume by the volume of the object. As used herein, a water content of a carrier means the water content of the carrier itself excluding a microorganism (e.g., before immobilizing a microorganism).

As used herein, "carrier" refers to a part for immobilizing a microorganism or the like which is installed in the gas phase reaction chamber of the present disclosure or constitutes a part of the wall of the gas phase reaction chamber of the present disclosure. A carrier may be composed of any material as long as it can immobilize a microorganism. A carrier can be preferably configured to comprise a small amount of moisture or allow moisture to permeate.

As used herein, "support of a carrier" refers to a part for immobilizing the carrier, which is also referred to as carrier retaining portion. A support of a carrier is used to immobilize the carrier in a gas phase reaction chamber or on the wall surface thereof such that at least a part of an immobilized microorganism is exposed to a gas phase.

As used herein, "liquid phase" refers to a space most of which is occupied by liquid among three forms of a substance consisting of gas, liquid, and a solid. The liquid phase as used herein also includes a space in which gas (e.g., an air bubble of a gaseous substrate) or a solid is dispersed in liquid.

As used herein, "gas phase" refers to a space most of which is occupied by gas among three forms of a substance consisting of gas, liquid, and a solid. The gas phase as used herein also includes a space in which liquid (e.g., water) is dispersed in gas.

As used herein, "continuous supply" of a substance means that a substance (e.g., gas (e.g., a substrate in a gas state and the like), liquid (e.g., water, a substrate in a liquid state, and the like)) is continuously delivered to a subject. As used herein, "intermittent supply" (also referred to as "discontinuous supply" herein) of a substance means that a substance (e.g., gas (e.g., a substrate in a gas state and the like), liquid (e.g., water, a substrate in a liquid state, and the like)) is intermittently (discontinuously) delivered to a subject. In one embodiment, continuously or intermittently delivering a substance in a gas state into a gas phase reaction chamber from outside through a pipe or an intake opening (e.g., gas supply section) is also continuous supply or intermittent supply of a substance to the gas phase in the gas phase reaction chamber. In one embodiment, continuously vaporizing a liquid substrate and continuously delivering a gaseous substrate into a gas phase reaction chamber through a pipe or an intake opening (e.g., gas supply section) is also continuous supply.

As used herein, a carrier being "immersed in water" refers to a state in which at least a part of the surface of the carrier on the gas phase reaction chamber side is immersed in water.

As used herein, "to the extent without drying up" refers to a state in which there is no dripping water outside the cell and only moisture originally contained in the cell is maintained. In this state, the surface of the cell may be wet, but the surface of the cell is not so wet that a water droplet falls.

As used herein, "partial open system" for conversion by a microorganism means a condition under which the microorganism is not exposed to a completely external environment but is disposed in some type of shield such as chambers, and a substance (e.g., gas (e.g., a substrate in a gas state and the like), liquid (e.g., water, a substrate in a liquid state, a nutrient, an inhibitor, an inducer, and the like)) is continuously or intermittently delivered to the microorganism from outside. Alternatively, "partial open system" means that a part of the chamber is open, through which exchange of a substance with the external environment continuously or intermittently occurs.

As used herein, "gas phase microorganism reaction system" refers to a configuration which executes the conversion of a gaseous substrate using a microorganism of the present disclosure, originally means a system or an organization for accomplishing an objective, and refers to a configuration in which a plurality of elements are systematically configured to affect each other and a plurality of various apparatuses communicate with each other as needed.

As used herein, "gas supply section" refers to a part which supplies a gaseous substrate to a gas phase in the gas phase microorganism reaction system of the present disclosure.

As used herein, "moisture supply system" refers to a part which supplies moisture to a carrier to which a microorganism is immobilized in the gas phase microorganism reaction system of the present disclosure.

As used herein, "gas discharge section" refers to a part which extracts gas from a gas phase reaction chamber in the gas phase microorganism reaction system of the present disclosure.

As used herein, "liquid discharge section" refers to a part which extracts liquid from a gas phase reaction chamber in the gas phase microorganism reaction system of the present disclosure.

As used herein, "kit" refers to a unit providing parts to be provided (e.g., the part of the gas phase microorganism reaction system of the present disclosure, a microorganism, a user manual and the like) which are generally separated into two or more segments. Preferably, a kit advantageously comprises an instruction or user manual describing how the provided parts (e.g., the part of the gas phase microorganism reaction system of the present disclosure, or a microorganism) or the like are used or how the parts should be operated.

As used herein, "program" is used in the meaning that is commonly used in the art, describes a process to be performed by a computer in order, is treated as a "product" according to the Japanese Patent Law, and may also be referred to as "program product" in order to clarify that it is perceivable or tangible. Every computer operates in accordance with a program. With a modern computer, a program is expressed as data and stored in a recording medium or a storage apparatus, or can be provided from a cloud.

As used herein, "recording medium" is a recording medium which stores a program for executing the present disclosure. A recording medium may be any medium as long as it can record a program, is computer readable, and can cause other equipment such as computers to execute and implement the program that was consequently read. For example, a recording medium can be, but is not limited to, ROM or HDD which can be internally stored, or an external storage apparatus such as a magnetic disc or a flash memory such as a USB memory.

As used herein, the term "about" refers to the indicated value plus or minus 10% unless specifically defined otherwise. When "about" is used for a temperature, it refers to the indicated temperature plus or minus 5°C. When "about" is used for a pH, it refers to the indicated pH plus or minus 0.5.

### (Preferred embodiments)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

In one aspect, the present disclosure provides conversion of a gaseous substrate by a microorganism. Any means for achieving said conversion is intended as the scope of the present disclosure. For example, even without an explicit description, a description relating to the condition in conversion of a gaseous substrate by a microorganism should be understood as simultaneously disclosing means in various forms for performing the present disclosure, such as a part for achieving said condition in a gas phase microorganism reaction system and a step for achieving said condition in a method. Although a gas phase microorganism reaction system can be described as a whole to facilitate understanding, a characteristic part of the system can also be the subject of the present disclosure. Further, although a part of a gas phase microorganism reaction system can be described with a connection relationship and/or a positional relationship to facilitate understanding, the part does not need to be actually constructed in the connection relationship and/or the positional relationship. Said part can be the subject of the present disclosure as long as it is configured to be able to be constructed in the connection relationship and/or the positional relationship. The conversion of a gaseous substrate by a microorganism of the present disclosure may be industrial gaseous substrate treatment, or may be domestic gaseous substrate treatment (e.g., a product for preventing foul odor in a toilet and the like).

In one aspect, the present disclosure provides a gas phase microorganism reaction system for converting a gaseous substrate by a microorganism. This gas phase microorganism reaction system comprises at least a gas phase reaction chamber which provides a gas phase space to convert a gaseous substrate into another substance, a carrier to which a microorganism is immobilized such that the microorganism is in direct contact with gas in the chamber, and a gas supply section which supplies the gaseous substrate to a gas phase of the gas phase microorganism reaction system. Operation of a gas phase microorganism reaction system may require an electric power source, or may not require an electric power source. A gas phase microorganism reaction system which requires an electric power source may be switched between an operation state and a non-operation state by switching a switch. In one embodiment, the gas phase microorganism reaction system of the present disclosure is a partial open system, wherein a gas phase reaction chamber in which a microorganism converts a gaseous substrate comprises a supply channel (e.g., a gas supply section or a moisture supply system) for continuous supply or intermittent supply of a substance (e.g., a gaseous substrate or moisture) from outside the gas phase reaction chamber into the gas phase reaction chamber. In one embodiment, the supply channel may be in gas communication and/or liquid communication with the gas phase reaction chamber in which a microorganism converts a gaseous substrate, or may be configured to be switchable between gas communication and/or liquid communication and a blocked state. In one embodiment, a gas phase microorganism reaction system comprises a gas discharge section and/or a liquid discharge section which are different from the supply channel. In one embodiment, a gas phase microorganism reaction system comprises a moisture supply system (e.g., including a moisture supply channel) for supplying moisture from outside a gas phase reaction chamber to a carrier.

In one aspect, the present disclosure provides a method for converting a gaseous substrate by a microorganism. This method comprises exposing at least a part of the surface on which the microorganism is immobilized on a carrier to a gas phase comprising the gaseous substrate. In one embodiment, the method of the present disclosure is performed in a partial opening system, wherein a reaction in which a microorganism converts a gaseous substrate is performed in a gas phase reaction chamber into which a substance (e.g., a gaseous substance or moisture) is supplied from outside the gas phase reaction chamber.

In one embodiment, a gaseous substrate is supplied to a gas phase, and the gaseous substrate is delivered to a microorganism via the gas phase. A gaseous substrate may be supplied to a gas phase by vaporization of liquid or sublimation of gas, or may be supplied in a gas state to a gas phase through a pipe or the like. In one embodiment, a gaseous substrate is continuously supplied or intermittently supplied to a gas phase. In one embodiment, the present disclosure (e.g., the system or the method of the present disclosure) can have a period during which a gaseous substrate is continuously supplied to a gas phase for a predetermined period, e.g., 1 minute or longer, 5 minutes or longer, 10 minutes or longer, 20 minutes or longer, 30 minutes or longer, 45 minutes or longer, 1 hour or longer, 5 hours or longer, 10 hours or longer, 20 hours or longer, 1 day or longer, 3 days or longer, 5 days or longer, or 7 days or longer. In one embodiment, the present disclosure (e.g., the system or the method of the present disclosure) can have a period during which a gaseous substrate is not supplied to a gas phase (supply of the gaseous substrate is interrupted), e.g., 1 second or longer, 10 seconds or longer, 30 seconds or longer, 1 minute or longer, 5 minutes or longer, 10 minutes or longer, 20 minutes or longer, 30 minutes or longer, 45 minutes or longer, 1 hour or longer, 2 hours or less, 5 hours or less, 12 hours or less, or 24 hours or less. In one embodiment, an airflow (e.g., an airflow in one direction) may be generated in a gas phase to actively deliver a gaseous substrate to a microorganism. In one embodiment, a gaseous substrate may be delivered to a microorganism by passive diffusion from outside air.

In one embodiment, supply of a gaseous substrate into a gas phase may be controlled. While any suitable amount of gaseous substrate may be supplied to a gas phase, for example, 0.001 mL/hr or greater, 0.01 mL/hr or greater, 0.1 mL/hr or greater, 0.5 mL/hr or greater, 1 mL/hr or greater, 5 mL/hr or greater, 10 mL/hr or greater, 50 mL/hr or greater, 100 mL/hr or greater, 500 mL/hr or greater, 1000 mL/hr or greater, 5000 mL/hr or greater, or 10000 mL/hr or greater of gaseous substrate can be supplied with respect to a volume of 1 L gas phase. In another embodiment (e.g., an embodiment of passive diffusion), the net amount of gas supplied to a gas phase is 0, wherein only internal diffusion of a substance may occur.

In one embodiment, a microorganism is immobilized to a carrier. Immobilizing a microorganism to a carrier enables a chemical reaction to be performed in a gas phase without liquid in which the microorganism is suspended. In one embodiment, a microorganism is immobilized on the surface of a carrier. In one embodiment, a microorganism is immobilized on the surface of a carrier which is exposed to a gas phase. In one embodiment, a carrier itself separates a gas phase reaction chamber from a water phase chamber and a structure in which the face of the carrier on which a microorganism is immobilized faces the gas phase reaction chamber side can be employed. In this case, since there is no portion at which the liquid phase and the gas phase are in direct contact with each other as compared to, for example, the format of floating a carrier between the liquid phase and the gas phase, it is considered that direct evaporation of liquid is suppressed and substance transfer between gas and liquid which is not via the microorganism layer is also suppressed, such that the efficiency of contact of a substrate or the like with the microorganism is improved. In one embodiment, a carrier is installed between a gas phase and a liquid phase. For example, when a carrier installed between a gas phase and a liquid phase can supply water to the surface of the carrier on the gas phase side but is water-penetrative to the extent that the gas phase side of the carrier is not immersed, a layered structure in which the gas phase and the liquid phase are alternately arranged with the carrier therebetween, or an assembled structure in which the gas phase and the liquid phase are disposed inside and outside each of a plurality of hollow columnar carriers can be formed, whereby the area per volume which can be utilized for a conversion reaction for a gaseous substrate can be improved.

In one embodiment, when a carrier is installed between a gas phase and a liquid phase, moisture in a liquid state is not supplied to the carrier from the gas phase side. For example, this can be achieved by employing a structure in which the carrier is surrounded by a liquid-non-permeable material and/or a structure in which a liquid-permeable material or a flow channel other than the carrier does not exist between a gas phase reaction chamber and a water phase chamber. In one embodiment, the thickness of a carrier which is installed between a gas phase reaction chamber and a water phase chamber can be, for example, 0.01 to 100 mm, 0.01 to 50 mm, 0.01 to 10 mm, 0.01 to 5 mm, 0.01 to 1 mm, 0.01 to 0.5 mm, 0.01 to 0.1 mm, 0.05 to 100 mm, 0.05 to 50 mm, 0.05 to 10 mm, 0.05 to 5 mm, 0.05 to 1 mm, 0.05 to 0.5 mm, 0.1 to 100 mm, 0.1 to 50 mm, 0.1 to 10 mm, 0.1 to 5 mm, 0.1 to 1 mm, 0.5 to 100 mm, 0.5 to 50 mm, 0.5 to 10 mm, 0.5 to 5 mm, 1 to 100 mm, 1 to 50 mm, 1 to 10 mm, 5 to 100 mm, 5 to 50 mm or the like. In one embodiment, a carrier comprises a plurality of tubular members. The plurality of tubular members may be bundled in close contact with each other to form a columnar unit, or may be disposed at an interval from each other in an outer tube to form a columnar unit comprising the plurality of tubular members and the outer tube. When a gas phase is formed inside the tubular members, it is considered that the tubular members constitute the wall of the gas phase reaction chamber, and when a liquid phase is formed inside the tubular members, it is considered that the tubular members constitute the wall of the water phase chamber. Further, when a gas phase is formed between the tubular members and the outer tube, it is considered that the tubular members and the outer tube constitute the wall of the gas phase reaction chamber, and when a liquid phase is formed between the tubular members and the outer tube, it is considered that the tubular members and the outer tube constitute the wall of the water phase chamber.

In one embodiment, a carrier is installed in a gas phase. In one embodiment, lengthening a flow channel from supply to discharge of a gaseous substrate can improve the conversion rate for a gaseous substrate (rate of a decrease in a discharged gaseous substrate relative to a supplied gaseous substrate) even with the same volume of a gas phase. In one embodiment, configuring (e.g., selection of a shape, a property of the surface, or the like) a flow channel such that the flow of supplied gas is a turbulent flow can improve the conversion rate for a gaseous substrate even with the same volume of a gas phase. In one embodiment, decreasing the volume of a gas phase with which an immobilized microorganism is not in contact as much as possible can improve the conversion rate for a gaseous substrate even with the same volume of a gas phase. In one embodiment, increasing the specific surface area of the microorganism immobilized-surface of a carrier in contact with a gas phase can improve the conversion rate for a gaseous substrate even with the same volume of a gas phase. In one embodiment, stirring gas in a gas phase can improve the conversion rate for a gaseous substrate even with the same volume of a gas phase, and a gas phase stirring mechanism may be used because energy required for stirring gas is much less than the energy required for stirring liquid.

In one embodiment, moisture is supplied to a carrier. As used herein, moisture means not only water in a liquid state but also a water molecule in any form such as moisture in a gas state (water vapor) or moisture in a solid state (ice), unless noted otherwise. In one embodiment, moisture is supplied to the surface of a carrier on which a microorganism is immobilized. This embodiment also includes a case where moisture is introduced from one face of a thick carrier, the moisture is caused to pass through the inside of the carrier, and the moisture is supplied to a microorganism immobilized to the other face. Passage of moisture inside the carrier may be passive transport utilizing gravity and a capillary phenomenon or the like, may be active transport via application of water pressure and an operation of centrifugal force or the like, or may utilize lift generated from water vaporization. In one embodiment, moisture is supplied to a carrier as a liquid flow. In one embodiment, moisture is supplied to a carrier via an aerosol state or a gas state (e.g., dispersed in a gas phase). In one embodiment, moisture in a gas state is condensed on a carrier, thereby supplying moisture to the carrier. For example, condensation of moisture is controlled by the material and/or the structure of a carrier, the temperature of a carrier and/or a gas phase, the amount of water in a gas phase, the amount of moisture supplied from a moisture supply system or the like.

In one embodiment, the water content of a carrier in the conversion of a gaseous substrate by a microorganism of the present disclosure can be 70% or less, 60% or less, 50% or less, 40% or less, 30% or less, 20% or less, 15% or less, 10% or less, 7% or less, 5% or less, or 3% or less. Many microorganisms need water for their survival or exerting their ability. In one embodiment, the water content of a carrier in the conversion of a gaseous substrate by a microorganism of the present disclosure can be 0.01% or greater, 0.05% or greater, 0.1% or greater, 0.5% or greater, or 1% or greater. In one embodiment, the water content of a carrier in the conversion of a gaseous substrate by a microorganism of the present disclosure can be 0.01 to 70%, 0.01 to 60%, 0.01 to 50%, 0.01 to 40%, 0.01 to 30%, 0.01 to 20%, 0.01 to 10%, 0.01 to 7%, 0.01 to 5%, 0.1 to 60%, 0.1 to 20%, 0.1 to 10%, 0.1 to 7%, 0.1 to 5%, 0.5 to 60%, 0.5 to 20%, 0.5 to 10%, 0.5 to 7%, 0.5 to 5%, 1 to 60%, 1 to 20%, 1 to 10%, 1 to 7%, 1 to 5%, 2 to 60%, 2 to 20%, 2 to 10%, 2 to 7%, 2 to 5%, 3 to 60%, 3 to 20%, 3 to 10%, 3 to 7%, or 3 to 5%. Meanwhile, a low water content can increase the frequency of contact of a microorganism with a gaseous substrate and increase the conversion speed for a gaseous substrate. Thus, control of the water content can improve the stability and/or the efficiency in the conversion of a gaseous substrate of the present disclosure. Particularly, when a gaseous substrate is continuously supplied, it can be advantageous to control the water content because loss of water from a carrier can easily occur. In one embodiment, a microorganism is immobilized to a carrier such that at least a part of the microorganism cell is in direct contact with a gas phase in a gas phase reaction chamber without being immersed in water.

In one embodiment, the water content of a carrier in the conversion of a gaseous substrate by a microorganism of the present disclosure can be controlled by adjustment of the amount of moisture present in a gas phase, selection of the material and/or the structure of the carrier (e.g., balance of hydrophilicity/hydrophobicity, utilization of a capillary phenomenon, or thickness of the carrier), design of a flow channel (including a flow channel which supplies moisture by a capillary phenomenon) which supplies moisture to the carrier, adjustment of the movement speed of the carrier, adjustment of the contact level between the carrier and a liquid phase and/or adjustment of water pressure, adjustment of the temperature of a gas phase and/or the carrier or the like.

In one embodiment, a supplement such as nutrient components utilized by a microorganism (e.g., a small molecule water-soluble substance such as organic acids, trace elements, salts, or amino acids) or agents regulating metabolism or the like (e.g., an inhibitor for a specific metabolic pathway or an inducer), components elevating the activity of the microorganism that is used, surfactants, dry protection agents, components for maintaining a microorganism for a long period of time, components suppressing generation of other microorganisms, excipients, or antioxidants may be delivered to a microorganism by transfer of moisture in a liquid state. For example, the flow channel of water to the location where the microorganism is present may be provided with a part which is in direct or indirect (e.g., via a pipe or a water-permeable material) contact with a supplement storage section (which may be in a solid state or may be prepared at a high concentration), or a liquid phase comprising a supplement may be brought into contact with a carrier to which the microorganism is immobilized. In this case, a water flow in a certain direction may be present or not present in the flow channel of water. The supplement can be delivered to the microorganism by diffusion as long as there is fluid communication from the supplement storage section to the location where the microorganism is present.

In one embodiment, a carrier is water-permeable or water-absorbent. Examples of the form of a water-permeable or water-absorbent carrier include, but are not limited to, filters, fibers, sponge, other porous bodies and the like. In one embodiment, a carrier is configured to allow water to permeate by a capillary phenomenon. For example, the water-permeability or the water-absorbability of a carrier can be adjusted by the quality of material and/or the structure of the carrier. In one embodiment, a carrier is made from a material having a quality and a surface structure with a contact angle with water of 0° to 90°, 10° to 90°, 30° to 90°, 50° to 90°, 70° to 90°, 0° to 70°, 10° to 70°, 30° to 70°, 50° to 70°, 0° to 50°, 10° to 50°, 30° to 50°, 0° to 30°, 10° to 30°, or 0° to 10°. In one embodiment, a carrier has a density with a weight of 0.01 to 2 g, 0.02 to 2 g, 0.05 to 2 g, 0.1 to 2 g, 0.2 to 2 g, 0.5 to 2 g, 0.01 to 1 g, 0.02 to 1 g, 0.05 to 1 g, 0.1 to 1 g, 0.2 to 1 g, 0.5 to 1 g, 0.01 to 0.5 g, 0.02 to 0.5 g, 0.05 to 0.5 g, 0.1 to 0.5 g, 0.01 to 0.2 g, 0.02 to 0.2 g, 0.05 to 0.2 g, 0.1 to 0.2 g, 0.01 to 0.1 g, 0.02 to 0.1 g, or 0.05 to 0.1 g per 1 cm³ of the carrier.

The material of a carrier is not particularly limited, as long as a microorganism can be immobilized. Examples thereof include carbon fiber (PAN based, pitch based, phenol resin based, and the like), glass fiber, cellulose fiber, polyethylene resin, polypropylene resin, polyurethane resin, polystyrene resin, polyvinyl chloride resin, polyvinyl acetate resin, polyvinyl alcohol resin, polyethylene glycol resin, acrylic resin, ceramics, silicon, metal, charcoal, activated carbon, nonwoven fabric, and composites thereof, and the like. Examples of the shape of a carrier include fan blade-like, hollow columnar (hollow round column-like, hollow hexagonal column-like, and the like), tubular, cubic, cuboidal, columnar, spherical, discoidal, sheet-like, membrane-like, bead-like, and the like.

A microorganism can be immobilized to a carrier using any suitable method. For example, a microorganism suspension may be made to pass through a filter carrier, a microorganism suspension may be sprayed onto a carrier, a carrier may be immersed in a microorganism suspension, or a microorganism may be physicochemically caused to bind to a carrier by a linker (e.g., a bifunctional reagent, an antibody, and the like). For techniques of immobilizing microorganisms, see, for example, "Biseibutsu Koteika Ho niyoru Haisui Shori [Wastewater treatment by microorganism immobilization method], (Edited and authored by Ryuichi Sudo, The Industrial Water Institute)", "Biseibutsu Koteika Ho niyoru Mizushori - Tantai Koteika Ho Hokatsu Koteika Ho, Seibutsu Kasseitan Ho (Atarashii Mizushori Shiriizu (1) [Water treatment by microorganism immobilization method - carrier immobilization method and comprehensive immobilization method, Biological activated carbon method (New water treatment series (1)]) (Kazuhiro Mochizuki, Katsutoshi Hori, Hideki Tachimoto (authors), NTS Inc.)", or the like. In one embodiment, a surface layer adhesive protein of a microorganism cell may be utilized. In this case, it is also useful to introduce and express the exogenous gene of an adhesive protein into a microorganism which is not adhesive. The amount of a microorganism to be immobilized to a carrier can be suitably set. For example, 0.1% by weight or greater, 0.5% by weight or greater, 1% by weight or greater, 5% by weight or greater, 10% by weight or greater, 50% by weight or greater, or 100% by weight or greater of microorganism (dry weight) may be immobilized relative to the carrier weight.

In one embodiment, a carrier may move. Since movement of a carrier can improve the frequency of contact of a microorganism with a gaseous substrate, the efficiency of conversion of the gaseous substrate can be improved. In one embodiment, movement of a carrier is active, wherein, for example, a movement mechanism for causing the carrier to move is used. The carrier may or may not be connected with the movement mechanism. For example, a movement mechanism which is not connected with a carrier can cause the carrier to move via electromagnetic force, wind power or the like. Examples of movement of a carrier include rotation movement and vibration movement. Examples of a power source for driving a movement mechanism include, but are not limited to, electric power (which is supplied from, for example, a solar power generation system such as solar panels, a battery, or a general electric power source), wind power, and the like. In one embodiment, the shape of a rotation mechanism can be rod-like (e.g., rotation axis) or platelike (e.g., discoidal). While a carrier is immobilized to a rotation mechanism in any manner, a carrier may be immobilized with an angle (e.g., almost perpendicularly) relative to the rotation mechanism in one embodiment. In one embodiment, any number of blades, for example, 1 to 100 blades, for example, 1, 2, 3, 4, 5, 6, 8, 10, 12, 16, 20 blades and the like, may be immobilized to a rotation axis. In one embodiment, a rotation mechanism may comprise at least a part of a moisture supply system, wherein moisture can be supplied to a carrier via the rotation mechanism. For example, a rotation mechanism is connected to a liquid reservoir optionally via a member made of a water-permeable material, whereby moisture can be supplied to a carrier. In general, when a chamber is a sphere, the flow of inside gas is uniform, which eliminates stagnation or the like of the flow at the corner. Further, when a chamber is a sphere, it is easier to simulate the flow of gas, scaling up or the like can be facilitated, and design of the structure for axial rotation (using, for example, vibration movement) is easier and more simple.

In one embodiment, movement of a carrier can cause transfer of water inside the carrier and/or on the carrier. In one embodiment, quick rotation movement or vibration movement of a carrier can remove, from the carrier, liquid moisture that is weakly bound to the carrier. In one embodiment, movement of a carrier can promote vaporization of water inside the carrier and/or on the carrier into a gas phase. When moisture in a liquid state leaves a carrier, the moisture can move in such a manner that moisture in a liquid state in fluid communication is drawn to the carrier. Thus, movement of the carrier can involve supply of a supplement to a microorganism. In one embodiment, movement of a carrier can promote transfer of moisture in a liquid state inside the carrier and/or on the carrier. For example, since rotation movement can generate centrifugal force, said movement can transfer water in the center of a carrier to outside and can thereby change the water content of the carrier part. In one embodiment, rotation movement or vibration movement of a carrier can be performed at about 1 to 10000 rpm, about 1 to 1000 rpm, about 1 to 100 rpm, about 10 to 10000 rpm, about 10 to 1000 rpm, about 10 to 100 rpm, about 100 to 10000 rpm, or about 100 to 1000 rpm.

In one embodiment, a product resulting from the conversion of a gaseous substrate by a microorganism of the present disclosure can be collected. The method for collecting a product is not particularly limited. For example, a product can be collected by being released or volatilized in a gas phase, being dissolved in liquid moisture, being adsorbed to a solid phase surface, or the like.

### (Parts)

In one embodiment, the conversion of a gaseous substrate by a microorganism of the present disclosure is performed using a gas phase microorganism reaction system which can have one or more parts. Thus, these one or more parts also remain within the scope of the present disclosure. Any part for performing the conversion of a gaseous substrate by a microorganism of the present disclosure is intended. Examples of the part include a carrier to which a microorganism is immobilized or for immobilizing a microorganism, a gas phase reaction chamber or a member for constructing the same, a support for immobilizing a carrier (e.g., in the gas phase reaction chamber or on the wall surface thereof such that at least a part of the immobilized microorganism is exposed to a gas phase) (also referred to as carrier retaining portion), a movement mechanism which causes the carrier to move, a power source which causes the movement mechanism to move, a gas supply section which supplies a gaseous substrate to a gas phase (including a liquid reservoir which generates a gaseous substrate by vaporization and a solid which generates a gaseous substrate by vaporization or sublimation), a moisture supply system which supplies moisture to the carrier (including a liquid reservoir), a chamber for containing a liquid phase (water phase chamber), a gas discharge section which extracts gas from the gas phase reaction chamber, a liquid discharge section which extracts liquid from the gas phase reaction chamber, a mechanism for collecting a product resulting from conversion of the gaseous substrate by the microorganism, and the like. While a system comprising a part having a plurality of functions can be described herein, the system may comprise a plurality of parts each having a different function, or one part may have a plurality of functions. For example, a system comprising a gas supply section and a moisture supply system may comprise a part which supplies a gaseous substrate and supplies moisture (e.g., **(9)** in Figure **7(B)**). Further, one or more functions may be exerted by combining a plurality of parts (members). A plurality of parts may be separately provided, may be provided with being integrally molded (e.g., 3D printing or injection modeling), or may be provided with being assembled. In one embodiment, one or more of a liquid discharge section, a gas supply section, and a gas discharge section is connected to a gas phase reaction chamber. A kit comprising one or more parts constituting the gas phase microorganism reaction system of the present disclosure is also intended in the present disclosure. Apart from the parts, the kit may comprise an instruction containing an explanation of a method for using the parts and/or an explanation of a method for operating the gas phase microorganism reaction system.

In one embodiment, the gas phase microorganism reaction system of the present disclosure and/or a part thereof may be controlled by a program. A program can be configured to implement with a computer a method for the conversion of a gaseous substrate by a microorganism of the present disclosure. In one embodiment, this program can implement with a computer a method for performing or controlling at least one of supplying a gaseous substrate to a gas phase, supplying moisture to a carrier, and causing the carrier to move. Further, the present disclosure also provides a recording medium storing the program of the present disclosure. The gas phase microorganism reaction system of the present disclosure may comprise such a recording medium.

### (Operation environment)

In the present disclosure, any gaseous substrate that can be converted by a microorganism can be used. In one embodiment, a gaseous substrate which is used in the conversion by a microorganism of the present disclosure includes methane, ethane, ethylene, acetylene, propane, propylene, formaldehyde, acetaldehyde, ammonium, carbon dioxide, nitrogen oxide, hydrogen sulfide, sulfur oxide, terpenes, methanol, ethanol, toluene, naphthalene, camphor, and paradichlorobenzene. In one embodiment, the objective of the conversion of the present disclosure is to remove harmful substance such as foul odor substance, allergenic substance, or caustic substance. In one embodiment, the objective of the conversion of the present disclosure is to produce a desired product. Those skilled in the art can suitably select a microorganism depending on the type of gaseous substrate.

In one embodiment, the conversion of a gaseous substrate of the present disclosure can produce a desired product. For example, the desired product includes, but is not limited to, alcohols (such as methanol), carboxylic acids, aldehydes, ketones, terpenes, geranic acids, and the like. In one embodiment, an aromatic component may be produced.

In the present disclosure, any microorganism capable of converting a gaseous substrate can be used. Those skilled in the art can suitably select a microorganism based on the type of gaseous substrate, and further optionally depending on the type of desired product. In addition to bacteria, yeast, microalga, mosses and the like can also be selected as a microorganism. A suitable water content can be selected depending on the microorganism that is used. Further, in addition to a wild-type strain, a genetically modified gene-recombinant microorganism, a molecular bred strain created by a synthetic biological method, a microorganism subjected to genome editing or the like can also be used as a microorganism.

In one embodiment, the conversion of a gaseous substrate by a microorganism of the present disclosure is performed by maintaining a microorganism in any temperature environment such as 0 to 100°C, 5 to 70°C, 10 to 50°C, 15 to 40°C, 20 to 35°C, 70°C or less, 60°C or less, 50°C or less, 40°C or less, 30°C or less, 25°C or less, 20°C or less, 15°C or less, 10°C or less, 5°C or less, 0°C or less, about 70°C, about 60°C, about 50°C, about 40°C, about 30°C, about 25°C, about 15°C, about 10°C, about 5°C, or about 0°C.

In one embodiment, the conversion of a gaseous substrate by a microorganism of the present disclosure may be performed along with any operation which results in assistance in survival of a microorganism that is used and/or improvement in the conversion efficiency such as irradiation with light or application of voltage.

### (General technology)

The molecular biological methodology, biochemical methodology, and microbiological methodology used herein are well-known and conventionally used in the art.

### (Note)

As used herein, "or" is used when "at least one or more" of the listed matters in the sentence can be employed. When explicitly described herein as "within the range" of "two values", the range also includes the two values themselves.

The present disclosure is described hereinafter based on Examples. The above descriptions and the following Examples are not provided to limit the present disclosure, but for the sole purpose of exemplification. Thus, the scope of the present invention is not limited to the embodiments and Examples specifically described herein and is limited only by the scope of claims.

### [Examples]

The Examples are described hereinafter. When required, organisms used in the following Examples were handled in compliance with the guidelines stipulated by the Nagoya University, regulatory agency, or the Cartagena Protocol. For reagents, the specific products described in the Examples were used. However, the reagents can be substituted with an equivalent product from another manufacturer (Sigma-Aldrich, Fuji Film, Wako Pure Chemical, Nacalai Tesque, R & D Systems, USCN Life Science INC, Kanto Chemical, Funakoshi, Tokyo Chemical Industry, Merck, or the like).

A carrier to which methane assimilating bacteria (Methylococcus capsulatus(Bath)) were immobilized was prepared in accordance with the following procedures.

A glass fiber filter (GF/F 47, GE Healthcare) was set on a filter holder, and a microorganism suspension that had been freeze-thawed once was made to pass through the filter by using a syringe. A washing solution was made to flow in a forward direction and a backward direction to remove a weakly bound cell. The liquid that was made to pass through the filter was all collected, and the absorbance of the collected liquid was measured and the amount of cells contained in the collected liquid was calculated. The amount of cells contained in the collected liquid was subtracted from the amount of cells contained in the cell suspension to calculate the weight of cells (dry weight) immobilized to the filter.

The methane concentration in the collected gas and the methanol concentration in the liquid phase were quantified using gas chromatography (GC 2014, Shimadzu) equipped with a hydrogen flame ionization detector and a 25% Sorbitol Gasport B (60/80) glass column (GL Science). Gas chromatography was operated under the following conditions.
Carrier gas: nitrogen
Flow rate: 40 mL/min
Column temperature: 100°C
Detector temperature: 150°C

### (Example 1)

The bioreactor (gas phase microorganism reaction system) shown in Figures **1** and **2** was assembled. Mixed gas of methane and air was made to flow from one gas supply line. The bioreactor is configured to have a long flow channel from the gas supply section to the gas discharge section. The operation was performed under atmospheric pressure. The bioreactor was configured such that the liquid phase would circulate at a flow rate of 30 mL/min. The initial capacity of the liquid phase was about 10 mL. A methane assimilating bacteria immobilized filter was disposed between a gas phase and a liquid phase such that the face on which the bacteria were immobilized would be on the gas phase side.

Mixed gas of methane and air (methane content of 20%) was supplied to this bioreactor installed with a Methylococcus capsulatus(Bath) immobilized carrier, and an experiment of decomposition of methane was conducted at 42°C. The methane concentration in the discharged gas was measured under various conditions of cell density (cell dry weight per gas phase volume), gas flow rate, and methane concentration in supplied gas, and the methane decomposition rate was calculated from the change over time in said concentration.

Figure **3** shows the results. Efficient continuous methane decomposition using the bioreactor was achieved.

Next, an experiment of methane decomposition and methane → methanol conversion was conducted when air comprising 20% methane was supplied at a flow rate of 0.1 mL/min. For the first 6 hours, a medium comprising only buffer or comprising formic acid as reducing power was supplied and only methane decomposition was caused. After the 6 hours, 1 µM of cyclopropanol, which is a methanol dehydrogenase inhibitor, was added and methanol was produced from methane, provided that the medium was exchanged for a fresh medium at 9 hours, and the concentration of cyclopropanol was further raised to 10 µM.

Figure **4** shows the result. Stable methane decomposition and efficient continuous methanol production were achieved by the bioreactor.

Further, decomposition of methane in the presence and absence of a liquid phase in the water phase chamber was observed using this bioreactor installed with the carrier to which Methylococcus capsulatus(Bath) was immobilized. Air comprising 22.8% (v/v) of methane was supplied at 42°C, the methane concentration in the gas supply section and the gas discharge section was measured in the presence and absence of a liquid phase and under various conditions of gas flow rate, and the methane decomposition rate was calculated.

Figure **5** shows the results. The methane concentration in the gas discharge section was constantly kept at 14.6% in the absence of a liquid phase while said methane concentration was kept at 19.4% in the presence of a liquid phase. The methane consumption rate in the absence of a liquid phase was about 2.5-fold of the rate in the presence of a liquid phase, indicating that the water content of a carrier can be important for efficient conversion of a gaseous substrate. Further, the methane decomposition rate was improved with an increase in the flow rate of gas under the condition of the absence of a liquid phase. This indicates that the flow rate of gas can affect the water content of the surface of the carrier and also affect the methane decomposition rate.

### (Example 2)

The bioreactor (gas phase microorganism reaction system) of Figures **6** to **9** was assembled. The details are shown in the explanation of Figure **7****.**

Neither water nor medium were placed in the chip in the following preliminary experiment. First, 2 mL of 12.9 mM phosphate buffer (1.83 g of Na₂HPO₄ and 1.76 g of KH₂PO₄ in 1 L of distilled water, at a pH of 7) was added, and moisture was transferred to the filter by a capillary phenomenon via tissue paper. Water was not added during the operation. A decomposition test was started in a state in which the filter was wetted first. The water content of the 10-blade fan was 53% (v/v) while the water content of the 20-blade fan was 58%. The bioreactor has a volume of about 150 mL. A filter to which Methylococcus capsulatus(Bath) was immobilized was used for the blades of the fan. This time, since water was not placed in the liquid reservoir of **(16)** of Figure **7(C)****,** and moisture was not supplied to the blades of the fan via **(15)** of Figure **7(C)** during the operation, the water content of the fan decreased to 20% or less at the end of the operation.

Decomposition of methane at 42°C which was continuously supplied by the bioreactor using the 10-blade fan or the 20-blade fan was observed. The fan was rotated at 100 rpm. The methane concentration in the discharged gas was measured under various conditions of the amount of supplied methane (mL/min), and the methane decomposition rate was calculated.

Figure **10** shows the results. Efficient continuous methane decomposition was achieved in both bioreactors. Especially, when the 20-blade fan was used, the methane decomposition rate was improved about 3-fold as compared to the case wherein the 10-blade fan was used.

Similarly, the methane concentration in the gas discharge section was measured when air comprising 20% (v/v) of methane was supplied at a flow rate of 8 mL/min.

Figure **11** shows the results. Stable continuous methane decomposition was achieved in both bioreactors.

### (Example 3)

The bioreactor (gas phase microorganism reaction system) comprising the parts of Figures **12** and **13** is assembled. Efficient and/or stable conversion of a gaseous substrate by a microorganism is achieved.

### (Example 4)

A genus *Acinetobacter* bacteria Tol5 strain subjected to genome editing to be able to control gene expression of the toluene decomposition pathway is applied to the bioreactor described in Figures **1** and **2** to convert gaseous toluene into methylcatechol. Stable consumption of toluene and production of methylcatechol are achieved.

### (Example 5)

A gas phase reaction chamber is filled with a polyurethane carrier to which a molecular bred strain of genus *Acinetobacter* bacteria Tol5, which is imparted with an ability to produce geranic acid by a synthetic biological method, is immobilized. Mixed gas of gaseous geraniol and air is continuously supplied from outside the chamber to cause a reaction of conversion into geranic acid. Discharge of gas comprising geraniol at a stable concentration from the outlet of the gas phase reaction chamber is confirmed, and collection of produced geranic acid through adsorption to the polyurethane carrier is achieved at the same time.

### (Example 6)

The bioreactor (gas phase microorganism reaction system) shown in Figure **15** was assembled. Mixed gas of methane and air was made to flow from one gas supply line. Unlike the bioreactor of Example 1, the flow channel is not spiral. In a similar manner to Example 1, a methane assimilating bacteria immobilized filter (immobilized bacteria amount: 9.95 g (dry weight)/m²) was disposed between a gas phase and a liquid phase such that the face on which the bacteria were immobilized would be on the gas phase side (Figure **16**), or disposed such that the face on which the bacteria were immobilized would be on the liquid phase side (Figure **17**), and mixed gas of methane and air (methane content of 20%) was supplied to conduct a methane → methanol conversion experiment. The gas flow amount was 3 mL/min, the medium circulating flow amount was 10 mL/min, and an NMS medium (KNO₃ 1.0 g/L; MgSO₄·7H₂O 1.0 g/L; CaCl₂·2H₂O 0.2 g/L; Na₂HPO₄ 0.497 g/L; KH₂PO₄ 0.39 g/L; Fe-EDTA 0.019 g/L; Na₂MoO₄·2H₂O 0.5 mg/L; FeSO₄·7H₂O 0.5 mg/L; ZnSO₄·7H₂O 0.4 mg/L; Na₂EDTA 0.25mg/L; H₃BO₃ 0.015 mg/L; CoCl₂·6H₂O 0.05 mg/L; MnCl₂4H₂O 0.02 mg/L; NiCl₂·6H₂O 0.01 mg/L) comprising 10 µM of cyclopropanol and 10 mM of sodium formate was used as the medium. In this regard, with the configuration of a conventional membrane reactor in which the methane assimilating bacteria immobilized filter was disposed such that the face on which the bacteria were immobilized would be on the liquid phase side as shown in Figure **17****,** microorganisms initially immobilized detached off and were suspended on the water phase side.

Figure **18** shows the result from disposing the filter such that the face on which the bacteria were immobilized would be on the gas phase side **(A)** or the liquid phase side **(B).** It was confirmed that utilization of bacteria in a gas phase can achieve more efficient methanol conversion as compared to the conventional configuration utilizing bacteria in a liquid phase.

Similarly, the methane assimilating bacteria immobilized filter was disposed between a gas phase and a liquid phase such that the face on which the bacteria were immobilized would be on the gas phase side (Figure **19**), and a methane → methanol conversion experiment was conducted. In the above experiment (Figure **18**), since the liquid medium was circulated with a Perista pump and a new medium was not supplied, the liquid portion is equal to under a batch operation. Meanwhile, in the present experiment (Figure **19**), the medium was circulated at the same flow amount (10 mL/min) as that of the above experiment (Figure **18**) and a fresh medium was also continuously supplied at 4 mL/hr at the same time, and discharged liquid comprising methanol was continuously collected at the same rate (4 mL/hr) as the medium supply rate. The gas flow amount was 0.2 mL/min.

Figure **20** shows the result. Stable and efficient continuous methane → methanol conversion was achieved by the bioreactor.

### (Example 7)

Decomposition of toluene was tested in a fan-type bioreactor. The experiment was conducted in accordance with the following procedures. Figure **21** shows the summary of the system in the present example.

Preparation of a toluene sample 1 mL of toluene was added dropwise to a 125 mL flask by using a pipette. Next, a rubber plug was attached to the two ports of the flask to seal the ports. The flask was then left standing for 1 hour at room temperature, and used as a sample to be introduced from the inlet of the bioreactor. Toluene vapor was at saturated vapor pressure at 25°C.

### Culture and immobilization of Tol5

Wild-type cells of *Acinetobacter* Tol5 (S.Ishii, J.Koki, H.Unno, K.Hori; Two Morphological Types of Cell Appendages on a Strongly Adhesive Bacterium, Acinetobacter sp. Strain Tol5, Appl.Environ.Microbiol.70, (2004) 5026-5029) were cultured in a 150 mL LB medium for 24 hours. All cells were collected and washed three times with deionized water by centrifugation at 6000 rpm for 10 minutes. A cell pellet was re-suspended in 10 mL of deionized water, and the suspension was added dropwise to a fan unit by using a pipette. Tol5 cells having a dry weight of 140 mg were collected. The cell density on the surface of the filter was 3 g (dry weight)/m².

### Construction of a fan-type bioreactor

Two members, i.e., a bottom cover with a gas inlet connector and a top cover with a gas outlet connector, were combined and screwed to assemble a gas chamber with a capacity of 150 mL. A fan unit was set in the gas chamber to assemble a fan-type bioreactor. In order to rotate the fan unit, a rotor was attached to the top cover of the fan-type bioreactor and driven by a battery. A PTFE gasket was used to connect each member in order to prevent gas leakage. A syringe plunger was attached to the rotation axis of the rotor and assembled with the fan unit (of which central axis is a syringe barrel). The fan unit was composed of 20 filter paper blades.

### Preparation of the fan-type bioreactor

After cells were immobilized to the fan unit, the fan unit was assembled to the axis of the rotor. The gas chamber was sealed, and 5 mL of deionized water was infused at the bottom of the bioreactor in order to maintain the humidity in the system saturated. A bioreactor comprising a fan unit without immobilized cells was also prepared as a control.

### Infusion of toluene vapor and operation of the bioreactor

A tube for infusing pure air from an air cylinder was connected to one port of the flask comprising toluene vapor, and the other port was connected to the inlet port of the fan-type bioreactor. The flow amount of air was controlled by a gas blender. Air was infused into the flask at a flow amount of 10 mL/min and mixed with toluene vapor, and the mixture thereof was infused into the bioreactor as an inlet gas sample. The toluene concentration in the inlet gas was 32.2 µM. The retention time of toluene vapor in the bioreactor was 12.5 minutes. The fan was rotated by the rotor at 25 rpm, thereby efficiently bringing toluene vapor into contact with the microorganisms immobilized to the fan and causing a reaction. This bioreactor operation was performed at room temperature.

### Acquisition and quantification of a gas sample

A gas sample (5 *µ* L) was collected from each of the inlet of the bioreactor and the outlet bioreactor and infused into gas chromatography-mass spectrometry (GC/MS) using an airtight syringe. Next, the toluene concentration was quantified by using a GC/MS system comprising an MS detector (MSD 5975; Agilent Technologies) connected to a GC system (GC7820A; Agilent Technologies, Santa Clara, California) equipped with an Rtx-200 capillary column (30 m × 0.32 mm × 0.5 um; RESTEC, Bellefonte, Pennsylvania). The split ratio and the flow amount of helium were set to be 10:1 and 2 mL/min, respectively. The operation program was started with an isocratic step at 90°C for 1 minute, followed by raising the temperature to the final temperature of 120°C at 25°C/min and monitoring ion fragments of m/z = 65 and m/z = 91 in a selected ion monitoring mode. A peak indicating toluene was detected at a retention time of 2.1 minutes. A peak area for calculation of the toluene concentration in a gas phase was manually measured.

The toluene removal rate was calculated by comparing the outlet toluene concentration of the control experiment (bioreactor without immobilized cells) to that of the test experiment (bioreactor with immobilized cells).

Figure **22** shows the result. When toluene vapor was supplied to the fan-type bioreactor at a retention time of 12.5 minutes, about 37% of toluene was continuously removed by *Acinetobacter* Tol5 cells, from which superior performance of the bioreactor was confirmed.

### (Note)

As described above, the present disclosure is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein.

The present application claims priority to Japanese Patent Application No. 2020-71961 filed to the Japan Patent Office on April 13, 2020. The entire content thereof is incorporated herein by reference.

### [Industrial Applicability]

The present disclosure provides efficient and/or stable conversion of a gaseous substrate using a microorganism and can thereby promote utilization of the microorganism for removal of unnecessary gas and/or acquisition of a desired product. Further, since the conversion of a gaseous substrate according to the present disclosure can be efficient and/or stable, said conversion can also contribute to reduction in the size of a gas phase microorganism reaction system.

## Claims

1. A gas phase microorganism reaction system comprising:
a gas phase reaction chamber comprising a gas supply section which continuously or intermittently supplies a gaseous substrate from outside;
a carrier which is installed in the gas phase reaction chamber or a carrier which constitutes at least a part of a wall of the gas phase reaction chamber, wherein the carrier is moisture-permeable or water-absorbent, but put in a state where any part of a surface on the gas phase side is not immersed in water, wherein a microorganism cell is immobilized to the carrier such that at least a part of the microorganism cell is in direct contact with a gas phase in the gas phase reaction chamber; and
a gas discharge section which is connected to the gas phase reaction chamber.

2. The gas phase microorganism reaction system of claim 1, further comprising a moisture supply system for supplying moisture from outside the gas phase reaction chamber to the extent without drying up of the microorganism cell that is immobilized to the carrier.

3. The gas phase microorganism reaction system of claim 2, wherein the moisture supply system is configured such that a water content of the carrier is 1 to 50% (v/v).

4. The gas phase microorganism reaction system of any one of claims 1 to 3, further comprising a mechanism which collects a product resulting from conversion of the gaseous substrate by the microorganism.

5. The system of any one of claims 1 to 4, wherein the gas supply section is configured to passively take in the gaseous substrate by diffusion from outside the gas phase reaction chamber.

6. The gas phase microorganism reaction system of any one of claims 1 to 5, wherein the carrier is a thin membrane.

7. The gas phase microorganism reaction system of any one of claims 1 to 6, further comprising a liquid discharge section which is connected to the gas phase reaction chamber.

8. The gas phase microorganism reaction system of any one of claims 2 to 7, wherein the moisture supply system is configured to supply the moisture to the carrier as a liquid flow.

9. The gas phase microorganism reaction system of claim 8, wherein the moisture supply system comprises a water phase chamber in which liquid flows, wherein the carrier is disposed between the water phase chamber and the gas phase reaction chamber, and wherein the carrier is disposed such that a surface of the carrier on which the microorganism is immobilized faces the gas phase reaction chamber side.

10. The gas phase microorganism reaction system of claim 9, wherein the reaction vessel comprises a plurality of gas phases and a plurality of liquid phases, the gas phases and the liquid phases being configured to be alternately layered.

11. The gas phase microorganism reaction system of claim 9 or 10, wherein the carrier comprises a plurality of tubular members, wherein the plurality of tubular members are bundled in close contact with each other to form a columnar unit, or the plurality of tubular members are disposed at an interval from each other in an outer tube to form a columnar unit comprising the plurality of tubular members and the outer tube, and wherein the plurality of tubular members and the outer tube constitute a wall of the gas phase reaction chamber or the water phase chamber.

12. The gas phase microorganism reaction system of any one of claims 1 to 8, further comprising a rotation mechanism which is connected to the carrier, wherein the rotation mechanism comprises a rotation axis to which the carrier is immobilized.

13. The gas phase microorganism reaction system of any one of claims 2 to 8, wherein the moisture supply system is configured to supply the moisture to the carrier by a capillary phenomenon.

14. The gas phase microorganism reaction system of any one of claims 2 to 8, wherein the moisture supply system is configured to supply the moisture to the carrier in an aerosol state or a gas state.

15. The gas phase microorganism reaction system of any one of claims 2 to 8, wherein the moisture supply system is configured such that the moisture in a gas state is condensed on the carrier.

16. The gas phase microorganism reaction system of any one of claims 12 to 15, further comprising a power source for driving the rotation mechanism.

17. A method for converting a gaseous substrate by a microorganism, the method comprising:
continuously or intermittently supplying a gaseous substrate from outside to a gas phase reaction chamber; and
exposing, in the gas phase reaction chamber, at least a part of a surface on which the microorganism is immobilized on the carrier to a gas phase in the gas phase reaction chamber.

18. The method of claim 17, further comprising supplying moisture to a microorganism loaded carrier from outside the gas phase reaction chamber.

19. The method of claim 17 or 18, which controls a water content of the carrier to 1 to 50%.

20. A kit comprising:
a carrier for immobilizing a microorganism or a carrier to which a microorganism is immobilized;
a gas phase reaction chamber or a gas phase reaction chamber member configured to be assembled to provide the gas phase reaction chamber;
a gas supply section which continuously or intermittently supplies a gaseous substrate from outside; and
a gas discharge section,
wherein the gas phase reaction chamber may be provided in a state where the gas phase reaction chamber comprises at least one of the carrier, the gas supply section, and the gas discharge section, or may be provided separately from the carrier, the gas supply section, and the gas discharge section,
wherein the gas phase reaction chamber is configured to contain the carrier, and
wherein the gas phase reaction chamber is configured such that a microorganism immobilized to the carrier is in contact with the gaseous substrate.

21. The kit of claim 20, further comprising a moisture supply system for supplying moisture to the carrier from outside the gas phase reaction chamber.

22. The kit of claim 20 or 21, further comprising a support which is configured to support the carrier.
